(19) Europäisches Patentamt
European Patent Office
Office européen des brevets

(11) **EP 1 265 071 A2**

(12) **EUROPEAN PATENT APPLICATION**

(43) Date of publication:
**11.12.2002 Bulletin 2002/50**

(51) Int Cl.[7]: **G01N 33/543**, C12Q 1/68

(21) Application number: **02011687.7**

(22) Date of filing: **03.06.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **05.06.2001 JP 2001168970**
**05.06.2001 JP 2001168984**

(71) Applicant: **FUJI PHOTO FILM CO., LTD.**
**Kanagawa-ken, 250-0193 (JP)**

(72) Inventors:
- **Inomata, Hiroko**
**Asaka-shi, Saitama 351-8585 (JP)**
- **Kojima, Masayoshi**
**Asaka-shi, Saitama 351-8585 (JP)**
- **Sudo, Yukio**
**Asaka-shi, Saitama 351-8585 (JP)**
- **Shinoki, Hiroshi**
**Asaka-shi, Saitama 351-8585 (JP)**
- **Seshimoto, Osamu**
**Asaka-shi, Saitama 351-8585 (JP)**

(74) Representative: **Albrecht, Thomas, Dr. et al**
**Kraus & Weisert,**
**Thomas-Wimmer-Ring 15**
**80539 München (DE)**

(54) **Reactive solid support and DNA fragment detection tool**

(57) According to the present invention, there is provided a reactive solid support having a porous substrate wherein the porous region has a fine pore diameter of about 2nm to about 1000nm, a porosity of about 10% to about 90% and a thickness of about 0.01μm to about 70μm, to the surface of which a group of vinyl sulfonyl groups or their reactive precursor groups are fixed by covalent bond via a linking group, respectively. According to the present invention, a probe of a nucleotide derivative or its analog nucleotide such as an oligonucleotide, a polynucleotide, or a peptide nucleic acid can be fixed in high density with high stability on the surface of a solid support having a porosity.

Fig.3

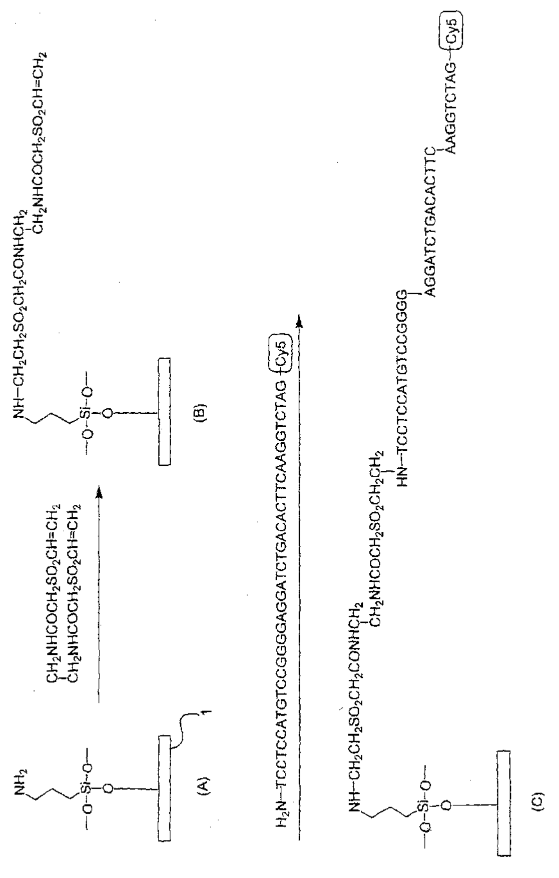



Printed by Jouve, 75001 PARIS (FR)

## Description

### Technical Field

The present invention relates to a detection tool useful for analyzing a structure of a biopolymer substance, and particularly relates to a detection tool useful for efficiently analyzing the expression, mutation, polymorphism and the like of a gene, wherein a large number of polymer substances originally from organisms or their analogs are aligned and fixed on the surface of a solid support having a porous substrate. The present invention particularly relates to a high density array type detection tool (DNA detection chip) useful for analyzing a base sequence of a DNA fragment sample wherein a number of nucleotide derivatives or their analogs are aligned and fixed to the surface of a solid support having a porous substrate, and a reactive solid support capable of advantageously being utilized for preparing the high density array type detection tool. Furthermore, the present invention relates to a chip useful for proteome analysis/proteomics wherein a protein or protein binding substance (except for nucleic acid) is fixed on the surface of the solid support, a method for detecting using said chip, and a method of manufacturing it.

### Background of the Invention

The technological development for efficiently analyzing gene functions of a variety of organisms has been rapidly progressed, and a detection tool called as a DNA chip where nucleotide derivatives such as a large number of DNA fragments or synthesized oligonucleotides or the like are fixed on the surface of a solid phase substrate has been employed in order to analyze the base sequence of the DNAs or DNA fragments. A molecule for detection such as a DNA or its fragments or a synthesized oligonucleotide like a nucleotide derivative bound to the surface of such a solid phase substrate is also referred to as a probe molecule. A representative DNA chip is a microarray in which a large number of probe molecules are aligned and fixed to a solid support such as a slide glass or the like. DNA chip related technologies relating to manufacturing of a DNA chip and its use are considered to be capable of also utilizable for detecting a biomolecules other than DNA. Therefore, these are expected to provide a new means for aiming at drug discovery research, development of a method of diagnosis of diseases or preventing the disease, or the like.

The DNA chip related technologies has been materialized since the fact that the method for determining the base sequence of a DNA by hybridization with an oligonucleotide was developed. Although this method could overcome the limitation of the method for determining the base sequence using gel electrophoresis, initially it did not come into practice.

Subsequently, by developing the DNA chip configured as described above and its preparation technique, the expression, mutation, polymorphism or the like of a gene has been capable of efficiently being examined in a short period of time. Specifically, a DNA fragment sample showing the complementarity to a DNA fragment or an oligonucleotide on the DNA chip prepared (which is also referred to as a target DNA fragment) is, in general, detected by utilizing the hybridization of the DNA fragment or the oligonucleotide on the DNA chip with the labeled DNA fragment sample.

In order to put the DNA chip preparation technique into practical use, a technology for aligning a large number of DNA fragments and oligonucleotides to surface of a solid support in high density and stable state is required.

As for a method for preparing a DNA chip, there are known a method for directly synthesizing oligonucleotide on the surface of the solid support (referred to as "on-chip method") and a method in which a DNA fragment or an oligonucleotide previously prepared is bound to the surface of the solid support. As for the on-chip method, a method for selectively synthesizing an oligonucleotide in the predetermined minute matrix region by combining use of a protective group selectively removable using photoirradiation, the photolithography technology used for fabricating a semiconductor device and a technology for synthesizing a solid phase (referred to as "masking technology") is representative.

As a method for binding and fixing a DNA fragment or an oligonucleotide previously prepared on the surface of the solid support, following methods are known corresponding to kinds of DNA fragments and kinds of solid supports.

(1) In the case where a DNA fragment to be fixed is a cDNA (complementary DNA synthesized by utilizing a mRNA as a template) or PCR products (DNA fragment obtained by amplifying the cDNA by a PCR method) , in general, the cDNAs or PCR products are dotted to the surface of the solid support which was surface-treated with a polycationic compound (poly-lysine, polyethyleneimine or the like) using a spotter device provided in a DNA chip preparation device, and are electrostatically bound to the solid support by utilizing the charge held in the DNA fragments. As a method for treating the surface of the solid support, a method of employing a silane coupling agent containing an amino group, an aldehyde group, an epoxy group or the like is also utilized. In the surface treatment using the silane coupling agent, since the amino group, the aldehyde group or the like is fixed on the surface of the solid support by covalent bond, it is more stably fixed on the surface of the solid support, as compared with the case of

surface treatment with a polycationic compound.

As a modified method for utilizing the charge of the DNA fragments described above, there is reported a method wherein PCR products modified with an amino group is suspended in SSC (standard salt-citric acid buffer solution), dotted to the surface of the sililated slide glass, incubated, and then treated with sodium boron hydride and then with heating. However, there is a problem that it is difficult to necessarily obtain the sufficient fixation stability of the DNA fragments by this fixation method. In DNA chip technologies, detection limit is important. Therefore, binding and fixing of DNA fragments on the surface of the solid support in a sufficient amount (i.e., in a high density) and in a stable state has a direct influence on increase of the detection limit of hybridization of DNA fragment probes and labeled nucleic acid fragments samples.

(2) In the case where an oligonucleotide (probe molecule) to be fixed is a synthesized oligonucleotide, there is known a method, in which an oligonucleotide into which a reactive group has been introduced is synthesized, then the oligonucleotide is dotted to the surface of the solid support surface-treated so as to previously form the reactive group, thereby binding and fixing the oligonucleotide to the surface of the solid support by covalent bond. For example, there are known a method in which an amino group-introduced oligonucleotide is reacted with the surface of the slide glass to which an amino group is introduced in the presence of PDC (p-phenylene diisothiocyanate), and a method in which an aldehyde group- introduced oligonucleotide is reacted with said slide glass. These two methods are more advantageous from the viewpoint that an oligonucleotide is stably bound and fixed to the surface of the solid support, as compared with the above-described method (1) for electrostatically binding by utilizing the charge of the DNA fragments. However, there are problems that in a method of performing the reaction in the presence of PDC, the reaction of PDC and an amino group-introduced oligonucleotide is slow, and in a method of using an aldehyde group-introduced oligonucleotide, the stability of Schiff base which is a reactive product is low (that is, hydrolysis is easily occurred).

In recent years, a technology employing an oligonucleotide analog which is referred to as PNA (peptide nucleic acid) instead of an oligonucleotide or a polynucleotide (also including a synthesized oligonucleotide or polynucleotide and a DNA molecule and DNA fragment, and a RNA molecule and RNA fragment) as a probe molecule of a DNA chip has also been proposed. As a method for fixing PNA to the solid phase substrate by covalent bond, a method of using the combination of avidin and biotin is also known (Japanese Unexamined Patent Publication No.H11-332595 gazette) . In this publication gazette, a technology utilizing a surface plasmon resonance (SPR) biosenser as the solid phase substrate is also described. Utilizing the DNA chip in which a probe molecule is fixed on the surface plasmon resonance biosenser, a DNA fragment bound to its surface via hybridization can be detected by utilizing the surface plasmon resonance phenomenon.

Moreover, as a substrate of the DNA chip, use of a charge coupled device (CCD) is also known (Nucleic Acid Research, 1994, Vol. 22, No. 11, pp. 2124-2125).

In Japanese Unexamined Patent Publication No. H04-228076 gazette (corresponding to U. S. Patent No. 5,387,505), a technology for isolating a target DNA is describe. In this technology, the target DNA having biotin molecule is bound to a substrate, the surface of which an avidins molecule is fixed on.

Japanese Patent Publication No.H07-43380 gazette (corresponding to U. S. Patent No.5,094,962) describes a detection tool used for ligand-receptor assay, that is, an analyzing tool that an receptor molecule is bound to surface of a microporous polymer particle having a reactively active group on its surface.

On the other hand, in recent years, since the genomic analysis has been almost completed, the "proteome/ proteomics" research, that provides essential information in order to finally understand meanings of the gene information and to simulate the life-activities of the cells, has been progressed. The term "proteome" means the all sets of proteins which are translated and produced in a specific cell, an apparatus and an organ, and the research field of high-level information analysis of chemical structure, total amount, expression period, modification after translation, formation of aggregation and the like are referred to as "proteomics".

The proteome research includes a profiling of proteins, an identification and precise analysis of proteins, a interaction network analysis and construction of a proteome data base, and is a field where these technologies are applied to the life science researches.

Among these, as the interaction network analysis method, a yeast two-hybrid method and a phage display method have been performed, and as a method of utilizing affinity capture, an immunoprecipitation method, a BIA-MA method, a column switching-mass spectrometry method and the like have been performed ("Proteome analysis method", pp. 163-211, published by Yodosha, Co., Ltd., 2000). However, any of these interaction network analyses listed above has not achieved a high throughput analysis.

The report has been made by Schreiber et al. on a protein microarray for a high throughput analysis of interaction of proteins (Science 289: 1760-1763, 2000). This is a method in which protein aqueous solution is dotted to the

slide glass having an aldehyde group, blocked with BSA solution, then reacted with the protein solution to carry out detection by a fluorescence scanner. In this case, there is a problem that the stability of Schiff base which is a reaction product between an aldehyde group and an amino group is low (usually, hydrolysis is easily occurred).

In addition to these, as a method of fixing protein to the solid phase, a method, in which a hydrophobic polypeptide is introduced into the end of the protein, is described in Japanese Patent Publication No.H07-53108 gazette.

In Japanese Patent No.2,922,040, a method of fixing an antibody protein with protein A molecule film is described.

In the International Publication WO00/61282, the description on a porous solid support is disclosed. The thickness from the supporting body is made in the range from 0.01 to 70 μm and the porosity is in the range from 10 to 90% by coating particles mainly made of inorganic substances. When this porous solid support is employed, there is a merit that a fixation amount of an organism polymer is increased because its surface area is enlarged.

Disclosure of the Invention

Objects of the present invention are to provide a reactive solid support capable of particularly advantageously being used for stably binding and fixing a previously prepared nucleotide derivative such as an oligonucleotide, a polynucleotide, or a peptide nucleic acid or their analogs in a high density state on the surface of the solid support, and to provide a detection tool for detecting DNA, RNA or their fragments having a specific base sequence portion, in which the nucleotide derivative or its analog has been bound and fixed on the reactive solid support.

Further objects of the present invention are to provide a chip in which at least one protein or protein binding substance is bound and fixed to a reactive solid support which can achieve rapid and stable binding and fixing, and to provide a detection method for detecting a specifically binding target substance by utilizing the chip.

The present invention provides the following (1) to (55).

(1) A reactive solid support having a porous substrate wherein the porous region has a fine pore diameter of about 2nm to about 1000nm, a porosity of about 10% to about 90% and a thickness of about 0.01μm to about 70μm, to the surface of which a group of vinyl sulfonyl groups or their reactive precursor groups are fixed by covalent bond via a linking group, respectively.

(2) The reactive solid support of the above (1), wherein the porous substrate is composed of an organic polymer.

(3) The reactive solid support of the above (1), wherein the porous substrate is composed of an inorganic substrate.

(4) The reactive solid support of the above (1), wherein the porous substrate comprises silicon, alumina or titanium.

(5) The reactive solid support of the above (1), wherein a linked body of the vinylsulfonyl group or its reactive precursor group and the linking group is represented by the following formula:

$$-L-SO_2-X$$

in the above-described formula, X represents $-CR^1=CR^2R^3$ or $-CHR^1-CR^2R^3Y$, each of $R^1$, $R^2$ and $R^3$ represents independently from each other an atom or a group selected from the group consisted of a hydrogen atom, an alkyl group having 1 to 6 carbon atoms, an aryl group having 6 to 20 carbon atoms, and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and a quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group.

(6) The reactive solid support of the above (5), wherein X represents a vinyl group represented by $-CH=CH_2$.

(7) The reactive solid support of the above (5), wherein L represents a linking group containing an atom of a bivalence or more except for carbon atom.

(8) The reactive solid support of the above (5), wherein L represents a linking group having a linking portion selected from the group consisted of -NH-, -S- and -O-.

(9) The reactive solid support of the above (5), wherein L represents a linking group represented by $-(L^1)_n-NH-(CR^1R^2)_2-$ or $-(L^1)_n-S-(CR^1R^2)_2-$ wherein $R^1$ and $R^2$ represents the same meanings as described above, $L^1$ represents a linking group, and n represents either 0 or 1.

(10) The reactive solid support of the above (5), wherein L represents a linking group represented by $-(L^1)_n-NHCH_2CH_2-$ wherein $L^1$ represents a linking group, and n represents either 0 or 1.

(11) The reactive solid support of the above (9), wherein $L^1$ represents a linking group containing a group represented by -OSi-, and n represents 1.

(12) The reactive solid support of the above (1), wherein said solid support is a substrate in a sheet shape selected from the group consisted of a glass substrate, a resin substrate, a glass substrate or a resin substrate surface-treated with a silane coupling agent and a glass substrate or a resin substrate having a covering layer on its surface.

(13) The reactive solid support of the above (12), wherein said solid support is a substrate in a sheet shape selected from the group consisted of a silicate glass substrate, a silicate glass substrate surface-treated with a silane coupling agent and a silicate glass substrate covered by an organic covering layer.

(14) A method of manufacturing the reactive solid support of the above (5) , wherein a disulfone compound represented by the following formula is brought into contact with a reactive solid support to the surface of which a reactive group is introduced:

$$X^1\text{-}SO_2\text{-}L^2\text{-}SO_2\text{-}X^2$$

in the above-described formula, each of $X^1$ and $X^2$ represents independently from each other $-CR^1=CR^2R^3$ or $-CHR^1-CR^2R^3Y$, each of $R^1$, $R^2$ and $R^3$ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and a quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and $L^2$ represents a linking group.

(15) The method of manufacturing a reactive solid support as recited in the above (14), in which the reactive group introduced to the surface of said solid support is an amino group, a mercapto group or a hydroxyl group.

(16) A manufacturing method of a solid support comprising a nucleotide derivative or its analog bound to the surface of the support via a linking group having a sulfonyl group, wherein a surface of a reactive solid support having a porous substrate on which each of a group of vinylsulfonyl group or its reactive precursor group is fixed by covalent bond, is contacted with a nucleotide derivative or its analog having a reactive group which is capable of reacting with said reactive group to form a covalent bond.

(17) The manufacturing method of the above (16), wherein said nucleotide derivative or its analog is selected from the group consisted of an oligonucleotide, a polynucleotide and a peptide nucleic acid.

(18) The manufacturing method of the above (16), wherein a reactive solid support where a linked body of a vinylsulfonyl group or its reactive precursor group represented by the following formula and a linking group is bound to and fixed on, is used as a reactive solid support having a porous substrate:

$$-L\text{-}SO_2\text{-}X$$

in the above-described formula, X represents $-CR^1=CR^2R^3$ or $-CHR^1-CR^2R^3Y$, each of $R^1$, $R^2$ and $R^3$ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and a quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group or a single bond.

(19) The manufacturing method of the above (18), wherein X represents a reactive group represented by $-CR^1=CR^2R^3$ wherein each of $R^1$, $R^2$ and $R^3$ represents the same meanings as described above.

(20) A solid support having a porous substrate, in which a nucleotide derivative or its analog obtained by a manufacturing method of the above (16) is bound and fixed to surface of the solid support.

(21) A method of binding and fixing a complementary oligonucleotide or polynucleotide, wherein the solid support

having a porous substrate to which the nucleotide derivative or its analog is bound as recited in the above (20) , is contacted with an oligonucleotide or a polynucleotide having complementarity to said fixed nucleotide derivative or its analog in the presence of an aqueous medium.
(22) The method of the above (21), wherein a detectable label is bound to said complementary oligonucleotide or polynucleotide.
(23) A solid support to which a oligonucleotide or a polynucleotide having complementarity is bound and fixed wherein, to the solid support having a porous substrate to which the nucleotide derivative or its analog is bound, as recited in the above (20) , a oligonucleotide or a polynucleotide having complementarity to said fixed nucleotide derivative or its analog is bound in a complementary manner.
(24) The solid support of the above (23), wherein a detectable label is bound to said oligonucleotide or polynucleotide having the complementarity.
(25) A method of identifying or screening a gene, wherein the solid support having a porous substrate, to the surface of which the nucleotide derivative or its analog is bound and fixed as recited in the above (20), or the solid support to which the complementary oligonucleotide or polynucleotide is bound and fixed as recited in the above (23), is utilized.
(26) A biological material chip, wherein A, which represents a residue of at least one protein or protein binding substance, is bound to a solid support having a porous substrate wherein the porous region has a fine pore diameter of about 2nm to about 1000nm, a porosity of about 10% to about 90% and a thickness of about 0.01μm to about 70μm, by covalent bond via a sulfonyl group as shown in the following formula (I):

Solid support-L-$SO_2$-X-A (I)
in the formula (I) , L represents a linking group; X represents -$CR^1(R^2)$-$CR^3(R^4)$-; each of $R^1$, $R^2$, $R^3$ and $R^4$ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and A represents a residue of a protein or protein binding substance except for nucleic acid.

(27) The chip of the above (26) , wherein said protein or protein binding substance bounded to the surface is an antibody, an antibody fragment, a ligand, an antigen, a hapten or a receptor.
(28) The chip of the above (27) , wherein said protein or protein binding substance bound to the surface is avidins.
(29) The chip of the above (28), in which avidins are an avidin, a streptoavidin or altered bodies thereof which are capable of forming a stable complex with a biotin.
(30) The chip of the above (26) , wherein said protein bound to the surface is a nucleic acid recognition protein.
(31) The chip of the above (30), in which said nucleic acid recognition protein is a double stranded DNA recognition protein.
(32) The chip of the above (31), wherein said double stranded DNA recognition protein is a double stranded DNA recognition antibody.
(33) The chip of the above (31), wherein said double stranded DNA recognition protein is a DNA transcription factor.
(34) The chip of the above (31), wherein said double stranded DNA recognition protein is a protein having a Zinc finger motif or a Ring finger motif.
(35) The chip of the above (26), wherein the porous substrate is composed of an organic polymer.
(36) The chip of the above (26), wherein the porous substrate is particle composed of an inorganic substance.
(37) The chip of the above (26), wherein the porous substrate comprises silicon, alumina or titanium.
(38) The chip of the above (26), wherein said solid support is a glass, a plastic, an electrode surface or a sensor chip surface.
(39) A method of detecting a target substance, comprising the steps of:

contacting the chip of the above (26) with a sample containing a target substance which specifically binds to a protein or a protein binding substance except for nucleic acid supported on the surface of said chip; and detecting formation of reciprocal binding between said protein or protein binding substance and said target substance.

(40) The method of detecting a target substance as recited in the above (39), wherein said target substance is labeled with at least one component capable of generating a detectable signal.
(41) The method of detecting a target substance as recited in the above (39), comprising a step of performing blocking processing of the chip with an aqueous solution of an amino acid, a peptide or a protein.
(42) The method of manufacturing the chip as recited in the above (26) comprising a step in which a solid support having a porous substrate which contains a vinylsulfonyl group or its reactive precursor group represented by the

following formula (II) on its surface is contacted with at least one protein or protein binding substance having a reactive group which forms a covalent bond by reacting with said vinylsulfonyl group or its reactive precursor group:

$$-L-SO_2-X' \quad (II)$$

in the above-described formula (II), L represents a linking group which binds $-L-SO_2-X'$ to a solid support; X' represents $-CR^1=CR^2(R^3)$ or $-CH(R^1)-CR^2(R^3)(Y)$; each of $R^1$, $R^2$ and $R^3$ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and Y represents a group which is substituted by a neucleophilic reagent or a group which is eliminated as a "HY" by a base

(43) The method of manufacturing a chip as recited in the above (42), wherein said protein or protein binding substance bound to the surface is an antibody, an antibody fragment, a ligand, an antigen, a hapten or a receptor.
(44) The method of manufacturing a chip as recited in the above (42), wherein said protein or protein binding substance bound to the surface is avidins.
(45) The method of manufacturing a chip as recited in the above (44), wherein avidins are an avidin, a streptoavidin or altered bodies thereof which are capable of forming a stable complex with a biotin.
(46) The method of manufacturing a chip as recited in the above (42), wherein said protein bound to the surface is a nucleic acid recognition protein.
(47) The method of manufacturing a chip as recited in the above (46) , wherein said nucleic acid recognition protein is a double stranded DNA recognition protein.
(48) The method of manufacturing a chip as recited in the above (47), wherein said double stranded DNA recognition protein is a double stranded DNA recognition antibody.
(49) The method of manufacturing a chip as recited in the above (47), wherein said double stranded DNA recognition protein is a DNA transcription factor.
(50) The method of manufacturing a chip as recited in the above (47), wherein said double stranded DNA recognition protein is a protein having a Zinc finger motif or a Ring finger motif.
(51) The method of manufacturing a chip as recited in the above (42), wherein said porous substrate is composed of an organic polymer.
(52) The method of manufacturing a chip as recited in the above (42), wherein said porous substrate is particle composed of an inorganic substance.
(53) The method of manufacturing a chip as recited in the above (42), wherein said porous substrate comprises silicon, alumina or titanium.
(54) The method of manufacturing a chip as recited in the above (42), in which a solid support is a glass, a plastic, an electrode surface or a sensor chip surface.
(55) The method of manufacturing a chip as recited in the above (42), comprising the steps of:

fixing at least one protein or protein binding substance to a solid support having a porous substrate by contacting said protein or protein binding substance to said solid support; and
performing blocking process of a free vinylsulfonyl group or its reactive precursor group located on the surface of said solid support with an amino acid, a peptide or a protein aqueous solution.

Brief Description of the Drawings

FIG. 1 is a schematic diagram showing a representative oligonucleotide fixed solid support of the present invention and a representative method of the present invention for fixing an oligonucleotide;
FIG. 2 is a schematic diagram showing the configuration of a protein chip, which is a representative embodiment of the present invention; and

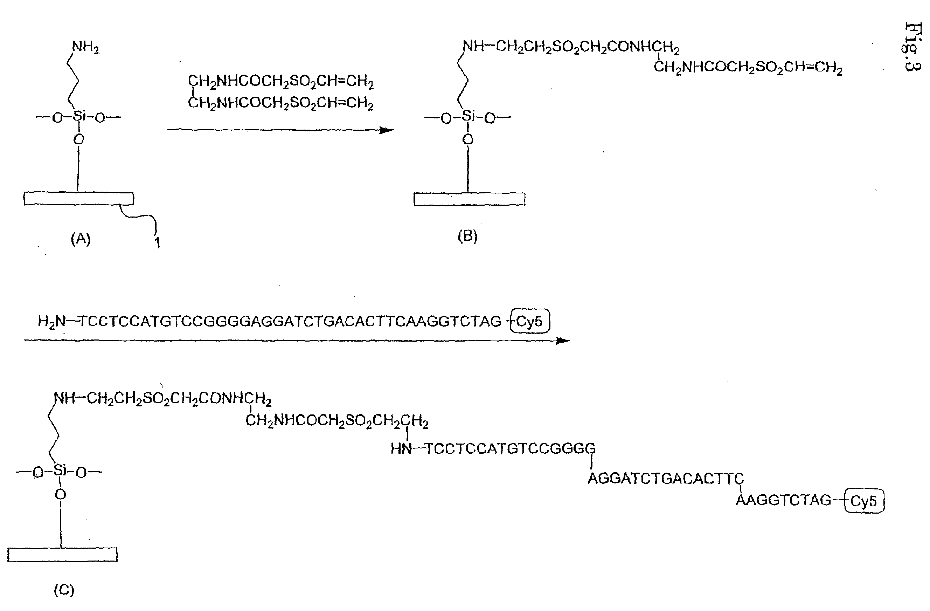

FIG. 3 is a schematic diagram showing a fixation method of Example 1;

Detailed Description of the Invention

A reactive solid support of the present invention has the configuration in which a group of vinyl sulfonyl groups or their reactive precursor groups are bound and fixed on the surface of the solid support having a porous substrate

via a linking group by covalent bond. The reactive solid support of the present invention can be manufactured by preparing the solid support having a porous substrate to the surface of which a reactive group has been previously introduced, and bringing this solid support into contact with a compound having a reactive group capable of forming a covalent bond by reacting with a reactive group provided to the surface of the support at one of end portions or nearby the end portion and having a vinyl sufonyl group or a reactive precursor group of the vinyl sufonyl group at the other end portion or nearby the end portion.

It is preferable that the solid support is a substrate having a flat and smooth surface. As a substance for the solid support, non-porous substances such as glass or plastic can be used.

Typical methods for forming a porous region includes a method by addition of material (for example, deposit) and a method by removal of material (for example, selective etching) .

In the method by addition, a porous region is formed on a surface of underlying support to increase an effective superficies. The porous region can be formed by the deposit of the following materials by using a solvent and catalyst as necessary. The porous region can be formed from, for example, a colloidal silica typically used in the sol-gel process, an organic silicon compound such as tetramethoxysilane, metal alkoxide, silyceskioxane or other silane, or combination thereof. As to the precursors of these, the morphology (fine pore diameter, porosity, thickness) of the porous region which is formed, can be controlled by suitably selecting parameters such as the composition, concentration and pH of the solution, aging period, and temperature. Also, an inorganic material other than the above (for example, aluminum or titanium based material) can be used in the same way as in the case of the above.

Alternatively, the porous region matrix can be cast. In the casting process, a material such as polymer is deposited with a matrix, and a porous structure having selected properties is generated by heat burning. The cast material may be any of a polymer such as polystyrene latex, polymer dissolved in a solution, or combination of these materials. The heat burning process is typically carried out by heating in the air, and can be carried out at a temperature from 150°C to a melting temperature (or a glass transition temperature) of the material which forms matrix of the porous layer. After the cast material is subjected to heat-burning, this matrix material can be burned. By changing the period and temperature of the burning process, various degrees of high density and fine pore property can be achieved.

The porous region can be formed on the surface of underlying support by various processes such as rotation coating, drenching coating, insufflation (aerosol), (physical or chemical) use of barrier which specifically deposit surface-deposited individual spots or coatings on channel, pad, spot or patterned surface.

The thickness of the porous region can be controlled by changing either or both of the precursor or the layer forming conditions. For example, the thickness can be controlled by the concentration of the reagent used. Also, the layer can be thickened by providing multiple deposition. During the coating of the agent to the successive layers, additional process may be carried out by baking the substrate before the coating of the subsequent reagent, to remove the solvent from the coating. Also, the thickness of the coating can be changed by controlling the speed of rotation or deposition. For example, thick coating can be prepared by decreasing the speed, and thin coating can be prepared by increasing the speed. Further, the thickness of the coating is effected by changing the speed of drawing from the drenching coating bath. Namely, thin coating is prepared by decreasing the speed, and thick coating is prepared by increasing the speed. The thickness of the coating is also effected by controlling the conditions of the solution. For example, when TMOS method is used, this solution can be subjected to gelation, thereby increasing the viscosity and therefore the thickness of the deposited layer.

The coating, particle or other components can be rotated on the surface of the substrate, and the substrate can be drenched with the solution containing the above-mentioned reagent. The reagent may be insufflated onto the surface of the substrate, or may be coated by other method. The substrate can be treated by interlacing the reagent in a grid, circular spot, area, cell, or any preferred configuration, and an area having a high porosity can be formed on whole surface of the substrate or only selected position.

The porosity of the substrate can be increased by utilizing a degradation method. For example, the porous region can be etched on the surface of the substrate (i.e., the surface of the material of the underlying substrate) . The surface may be prepared as etched glass such as a phase separating sodium borosilicate glass. In a particular embodiment of the degradation method for forming the porous substrate, fine pore diameter can be controlled by the cooling period and the temperature of the gradual cooling step which is carried out before the etching. Longer period of gradual cooling and higher temperature increase the fine pore diameter. The depth of the porous region can also be controlled by the etching parameter (solution concentration, composition, period, and the like) based on the substrate material.

If the porous material itself is porous as in the case of porous ceramics, porous activated carbon, woven fabric, non-woven fabric, filter paper, membrane filter or the like, the porous material can be sprayed onto the solid support or can be attached by using an adhesive. Also, the precursor of the porous material may be coated on the solid support, and the porosity can be made on the surface of the solid support. The typical examples include a sol-gel reaction. The general methods include the use of colloidal silica, but are not limited thereto.

As for an organic polymer which constitutes a porous substrate, monosaccharides such as glucose or fructose

or their derivatives, polysaccharides such as dextran, amylose or starch or their derivatives, for example, carboxymethyl starch, and high molecular polymers such as PVA, polyacrylic acid, cellulose, carboxymethyl cellulose, polyacetal and the like can be listed.

It is desirable to perform the covering treatment on the surface of the solid support having a porous substrate by a polymer containing an amino group such as a polycationic compound on side chain (for example, poly-L-lysine, polyethyleneimine, polyalkylamine or the like is preferable, and poly-L-lysine is more preferable) in order to bind and fix bifunctional reactive compounds such as divinyl sulfone compound or the like by covalent bond (in this case, the reactive group introduced to the surface of the solid support is an amino group). Alternatively, the surface of the solid support may be brought into contact and treated with a surface treatment agent having a reactive group such as a silane coupling agent which reacts with the surface of the solid support and a reactive group such as an amino group.

In the case where the covering treatment is performed with a polycationic compound, an amino group or a mercapto group is introduced to the surface of the solid support having a porous substrate by electrostatically binding between the polymer compound and the surface of the solid support. On the other hand, in the case where the surface treatment is performed by a silane coupling agent, the amino group or mercapto group stably exists on the surface of the solid support since it is bound and fixed to the surface of the solid support by covalent bond. In addition to an amino group or a mercapto group, an aldehyde group, an epoxy group, a carboxyl group or a hydroxyl group may be also preferably introduced.

As a silane coupling agent having an amino group, it is preferable to use γ -aminopropyltriethoxy silane, N-β (aminoethyl)- γ -aminopropyltrimethoxy silane, or N- β (aminoethyl)- γ -aminopropylmethyldimethoxy silane, and particularly it is preferable to use γ-aminopropyl triethoxy silane.

The treatment with a silane coupling agent may be performed in combination with the treatment using a polycationic compound. Using this method, an electrostatical interaction between a hydrophobic- or a low hydrophilic-solid support and DNA fragments can be promoted. A layer consisted of a hydrophilic polymer having a charge or the like or a layer consisted of a crosslinking agent may be further provided on the surface of the solid support treated by a polycationic compound. As a result of providing such a layer, the height of the convex and concave portions of the solid support treated by the polycationic compound can be reduced. Depending on the types of the solid supports, it is possible that a hydrophilic polymer is contained in the support, and the solid support subjected to such a treatment can be also preferably used.

On the surface of the usually utilized solid support for a DNA chip, a large number of regions previously fractioned or expected are set and provided, and in each region, as described above, a reactive group which is capable of reacting with bifunctional reactive compound such as divinyl sulfone compound or the like has been previously introduced. A reactive group such as the above-described amino group, mercapto group or hydroxyl group or the like is provided on the surface of each region of the solid support which is to be employed. However, on a solid support not having such a reactive group, as described above, the introduction of a reactive group is performed by surface treatment using a silane coupling agent, or by utilizing a method of coating and covering a polymer or the like having a reactive group such as an amino group on side chain on the surface of the solid support.

In the solid support equipped with a reactive group, as a result of bringing it into contact with a bifunctional reactive compound such as divinyl sulfone compound or the like, the reactive group and bifunctional reactive compound are reacted to form a covalent bond, the reactive group portion of the solid support is extended, and a reactive chain having a vinyl sulfonyl group or its reactive precursor group at its end or nearby the end is formed, thereby providing a reactive solid support of the present invention.

In a reactive solid support of the present invention, a linked body of a vinyl sulfonyl group or its reactive precursor group and a linking group introduced on the surface of the solid support is desirably a linked body represented by the following formula (1):

$$-L-SO_2-X \qquad (1)$$

In the above-described formula (1), X represents $-CR^1=CR^2R^3$ or $-CHR^1-CR^2R^3Y$ (reactive precursor group) . Each of $R^1$, $R^2$ and $R^3$ represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms. Examples of an alkyl group having 1 to 6 carbon atoms include a methyl group, an ethyl group, an n-propyl group, an n-butyl group, and an n-hexyl group. A methyl group is particularly preferable. Aryl groups include a phenyl group and a naphthyl group. It is preferable that each of $R^1$, $R^2$ and $R^3$ represents a hydrogen atom, respectively.

Y represents a group which is substituted by a nucleophilic reagent such as -OH, $-OR^0$, -SH, $NH_3$, $NH_2R^0$ (wherein $R^0$ represents a group such as alkyl group except for hydrogen atom) , or a group which is eliminated as "HY"

by base. Examples thereof include a halogen atom, $-OSO_2R^{11}$, $-OCOR^{12}$, $-OSO_3M$, or a quaternary pyridinium group ($R^{11}$ represents an alkyl group having 1 to 6 carbon atoms, an aryl group having 6 to 20 carbon atoms, or an aralkyl group having 7 to 26 carbon atoms in total containing an alkyl chain having 1 to 6 carbon atoms; $R^{12}$ represents an alkyl group having 1 to 6 carbon atoms or halogenated alkyl group having 1 to 6 carbon atoms; M represents a hydrogen atom, an alkaline metal atom, or an ammonium group) .

L represents a bivalent or more than bivalent linking group for linking the solid support or a linking group binding to the solid support with the above-described $-SO_2$-X group. However, L may be a single bond. Examples of the bivalent linking groups include an alkylene group having 1 to 6 carbon atoms, an aliphatic cyclic group having 3 to 16 carbon atoms, an arylene group having 6 to 20 carbon atoms, a heterocyclic group having 2 to 20 carbon atoms containing 1 to 3 hetero atoms selected from the group consisted of N, S and P, a group containing one group or the combination of a plurality of groups selected from the group consisted of -O-, -S-, -SO-, $-SO_2-$, $-SO_3-$, $-NR^{11}-$, -CO- and their combinations are preferable. $R^{11}$ is preferably a hydrogen atom, an alkyl group having 1 to 15 carbon atoms, an aryl group having 6 to 20 carbon atoms, or an aralkyl group having 7 to 21 carbon atoms containing an alkyl group having 1 to 6 carbon atoms, and more preferably a hydrogen atom or an alkyl group having 1 to 6 carbon atoms, and particularly preferably a hydrogen atom, a methyl group or an ethyl group.

In the case where L represents a group containing the combination of two or more of the groups selected from the group consisted of $-NR^{11}-$, $-SONR^{11}-$, $-CONR^{11}-$, $-NR^{11}COO-$, and $-NR^{11}CONR^{11}-$, these $R^{11}$ may bind each other to form a ring.

An alkyl group of $R^{11}$, an aryl group of $R^{11}$ and an aralkyl group of $R^{11}$ may have a substituent. Such substituents include an atom or a group selected from the group consisted of a hydroxyl group, an alkoxy group having 1 to 6 carbon atoms, an alkenyl group having 1 to 6 carbon atoms, a carbomoyl group having 2 to 7 carbon atoms, an alkyl group having 1 to 6 carbon atoms, an aralkyl group having 7 to 16 carbon atoms, an aryl group having 6 to 20 carbon atoms, an sulfamoyl group (or its Na salt, K salt or the like) , a sulfo group (or its Na salt, K salt or the like) , a carboxylic acid group (or its sodium salt, potassium salt or the like), a halogen atom, an alkenylene group having 1 to 6 carbon atoms, an arylene group having 6 to 20 carbon atoms, sulfonyl group and their combinations.

The preferable examples of the above-described "-X" group will be indicated below. Moreover, examples of a group which can be used as "$-L-SO_2-X$" will be indicated as described later.

(X1) $-CH{=}CH_2$

(X2) $-CH_2CH_2-Br$

(X3) $-CH_2CH_2-Cl$

(X4) $-CH_2CH_2-OSO_2CH_3$

(X5) $-CH_2CH_2-OSO_2C_6H_5$

(X6) $-CH_2CH_2-OSO_2C_6H_4-CH_3$

(X7) $-CH_2CH_2-OSO_3Na$

(X8) $-CH_2CH_2-OSO_3K$

(X9) $-CH_2CH_2-OCOCH_3$

(X10) $-CH_2CH_2-OCOCF_3$

(X11) $-CH_2CH_2-OCOCHCl_2$

(X12)

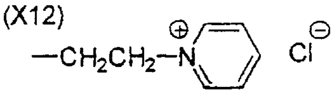

(X13)

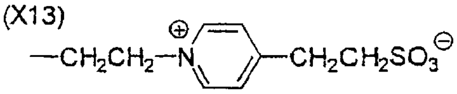

(X14)

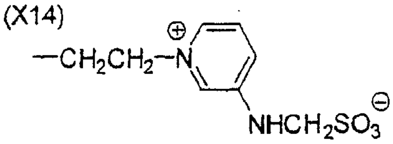

(X15)

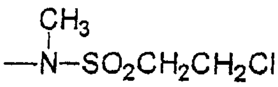

(X16)

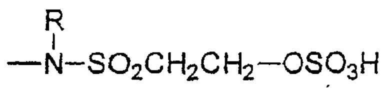

(X17)

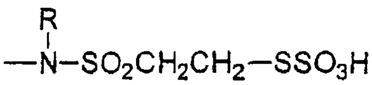

(X18)

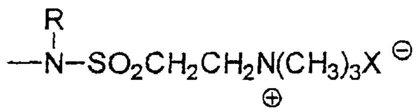

(X19)

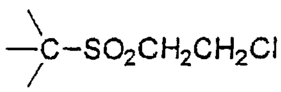

(X20)

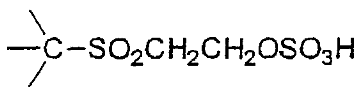

(X21)

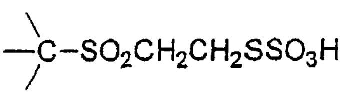

(X22)

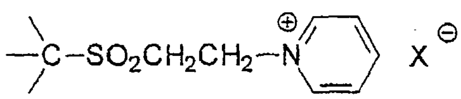

(X23)

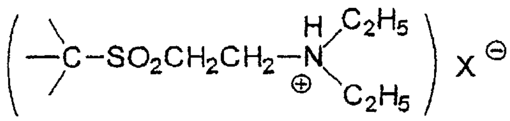

(X24)

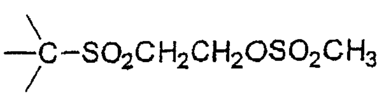

(X25)

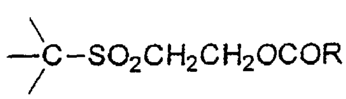

(X26)

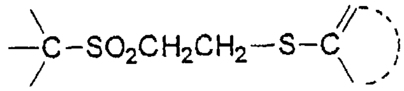

(X27)

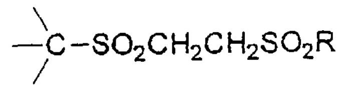

(X28)

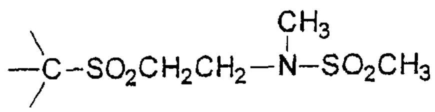

(X29)

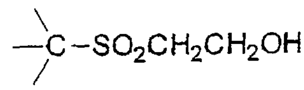

In the above-described examples, it is preferable that "-X" represents (X1), (X2), (X3), (X4), (X7), (X8), (X13) or (X14). It is more preferable that "-X" represents (X1) or (X2). It is particularly preferable that "-X" represents a vinyl group represented by (X1).

Preferable examples of L will be indicated below. "a" represents an integer of 1 to 6, preferably 1 or 2, and particularly preferably 1. "b" represents an integer of 0 to 6, and preferably either 2 or 3.

(L1)

$$-(CH_2)_a-$$

(L2)

$$-(CH_2)_a-O-(CH_2)_a-$$

(L3)

$$-(CH_2)_a-CONR^{11}-(CH_2)_b-NR^{11}CO-(CH_2)_a-$$

(L4)

$$-(CH_2)_a-SO_2-(CH_2)_a-$$

(L5)

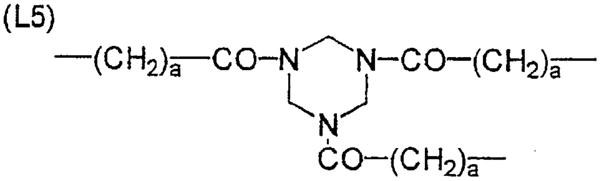

(L6)

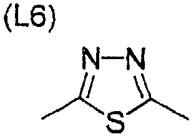

(L7)

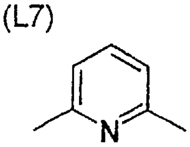

As for L, in addition to the above-described bivalent linking groups, a group that a hydrogen atom of the alkylene group in the above-described formula is substituted with a $-SO_2CH{=}CH_2$ group is also preferable.

As for a bifunctional reactive compound utilized for obtaining a solid support to which a vinyl sulfonyl group represented by the foregoing formula (1) or its reactive precursor group is fixed by covalent bond, a disulfone compound represented by the following formula (2) can be advantageously utilized.

$$X^1\text{-}SO_2\text{-}L^2\text{-}SO_2\text{-}X^2 \qquad (2)$$

[In the above-described formula, each of $X^1$ and $X^2$ independently from each other represents $-CR^1=CR^2R^3$ or $-CHR^1-CR^2R^3Y$ (reactive precursor group) ; each of $R^1$, $R^2$ and $R^3$ independently from each other represent an atom or a group selected from the group consisted of a hydrogen atom, an alkyl group having 1 to 6 carbon atoms, an aryl group having 6 to 20 carbon atoms, and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and ammonium group; and $L^2$ represents a linking group].

Specifically, a reactive solid support of the present invention can be easily manufactured by bringing a disulfone compound represented by the above-described formula (2) into contact with the above mentioned solid support, for example in the aqueous atmosphere.

The representative examples of disulfone compound preferably used in the present invention will be shown in the followings. It should be noted that a disulfone compound might be used by mixing two types or more.

(S1)

$$H_2C=CH-SO_2-CH_2-SO_2-CH=CH_2$$

(S2)

$$H_2C=CH-SO_2-CH_2OCH_2-SO_2-CH=CH_2$$

(S3)

$$H_2C=CH-SO_2-CH_2CH_2CH_2-SO_2-CH=CH_2$$

(S4)

$$H_2C=CH-SO_2-CH_2CH(OH)CH_2-SO_2-CH=CH_2$$

(S5)

$$H_2C=CH-SO_2-CH_2CONHCH_2CH_2NHCOCH_2-SO_2-CH=CH_2$$

(S6)

$$H_2C=CH-SO_2-CH_2CONHCH_2CH_2CH_2NHCOCH_2-SO_2-CH=CH_2$$

(S7)

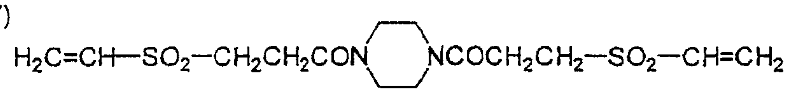

(S8)

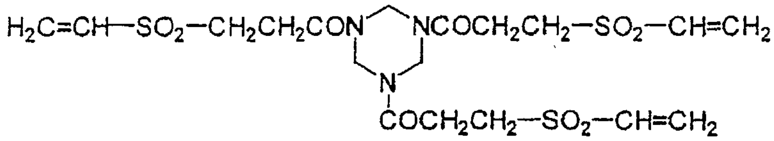

(S9)

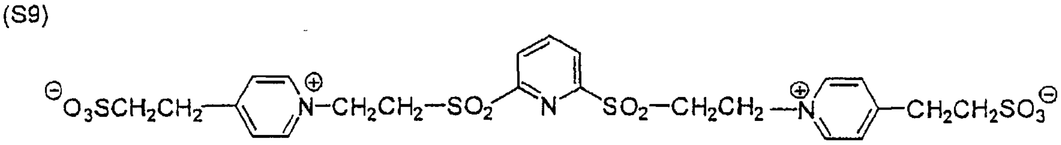

(S10)

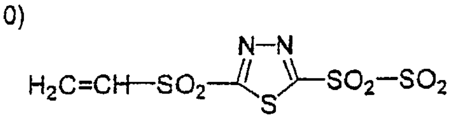

(S11)

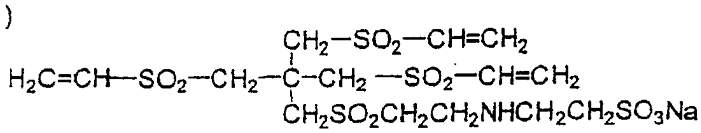

(S12)

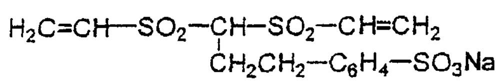

(S13)

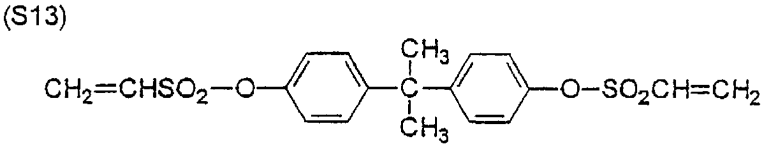

(S14)

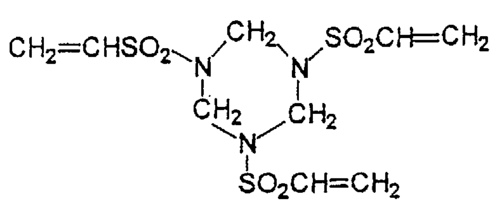

(S15)

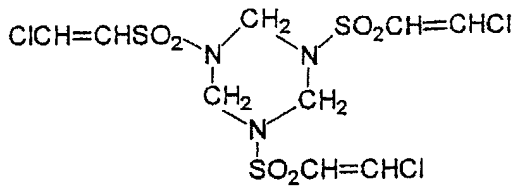

Representative examples of disulfone compound represented by the above-described formula (2) include 1,2-bis(vinylsulfonylacetamide)ethane [corresponding to the above-described S1].

As for a method for synthesizing a disulfone compound used in the present invention, the details have been described in a variety of gazettes, for example, such as Japanese Patent Publication No.S47-2429, Japanese Patent Publication No.S50-35807, Japanese Unexamined Patent Publication No.S49-24435, Japanese Unexamined Patent Publication No.S53-41551, Japanese Unexamined Patent Publication No.S59-18944 or the like.

In order to prepare a detection tool (in general, referred to as a DNA chip) for detecting by fixing a polynucleotide or an oligonucleotide originally from the nature such as DNA, RNA, DNA fragments, RNA fragments or the like by utilizing the reactive solid support obtained as described above, a method for bringing the above-described reactive solid support into contact with a nucleotide derivative or its analog equipped with a reactive group such as amino group which reacts with a vinyl sulfonyl group or its reactive precursor group on the above-described support surface to form a covalent bond is utilized. That is to say, in this way, a detection tool equipped with a probe molecule of the desired nucleotide derivative or its analog (what is called a DNA chip) can be prepared.

A vinyl sulfonyl group or its reactive precursor group bound via covalent bond to the surface of a solid phase substrate of the present invention can be readily preserved in a stable state since it has a high resistance to the hydrolysis, and can form a stable covalent bond by rapidly reacting with a nucleotide derivative or a reactive group of its analog in which amino group has been previously provided or a reactive group such as amino group has been introduced.

Representative examples of a nucleotide derivative and its analog used as a probe molecule include an oligonucleotide, a polynucleotide, and a peptide nucleic acid. These nucleotide derivatives or their analogs may be originally from the nature (DNA, DNA fragment, RNA or RNA fragment) , or may be a synthetic compound. Moreover, nucleotide derivatives or its analog include a variety of analogous compounds such as what is called a LNA having a crosslinking group at its sugar unit portion (J. Am. Chem. Soc. 1998, 120:13252-13253) are included.

In the case where DNA fragments are used as a probe molecule, these are divided into two types depending on purposes . In order to examine the expression of a gene, it is preferable to use a polynucleotide such as cDNA, one portion of cDNA, or EST. Functions of these polynucleotides may be unknown, but in general, these are prepared by amplifying cDNA library, genomic library, or the total genome as a template on the basis of the sequences registered on a data base by a PCR method (hereinafter, referred to as "PCR product"). Ones not amplified by a PCR method can be also preferably used. In order to examine the mutation and polymorphism of a gene, it is preferable to synthesize a variety of oligonucleotides corresponding to mutation and polymorphism based on the known sequence which is to be the standard, and use them. Furthermore, in the case where the purpose is to analyze the base sequence, it is preferable to synthesize $4^n$ (n represents the length of the base) species of oligonucleotides and use them. As for the base sequence of a DNA fragment, it is preferable that its sequence has been previously determined by a general base sequence determination method. The size of the DNA fragment is preferably from dimmer to 50-mer, and particularly preferably from 10- to 25-mer.

On one end of a nucleotide derivative such as an oligonucleotide and a DNA fragment, or its analog, a reactive group which forms a covalent bond by reacting with the foregoing vinyl sulfonyl group or its reactive precursor group is introduced. Such reactive groups include an amino group, an imino group, hydrazino group, carbomoyl group, hydrazinocarbonyl group or carboxyimido group, and the amino group is particularly preferable. The reactive group is usually bound to an oligonucleotide or a DNA fragment via a crosslinker. As the crosslinker, for example, an alkylene group or a N-alkylamino-alkylene group is utilized, and a hexylene group or a N-methylamino-hexylene group is preferable, and a hexylene group is particularly preferable. It should be noted that since a peptide nucleic acid (PNA) has an amino group, usually it is not necessary to introduce another reactive group.

Bringing a nucleotide derivative or its analog having a reactive group into contact with a reactive solid support is usually carried out by dotting the aqueous solution of the nucleotide derivative or its analog to the surface of the reactive solid support. Concretely, it is preferable that after an aqueous solution is prepared by dissolving or dispersing the nucleotide derivative or its analog having a reactive group in an aqueous medium, the aqueous solution is pipetted

into 96 wells or 384 wells plastic plate, and the pipetted aqueous solution is dropped on the surface of the solid support using a spotter device or the like.

In order to prevent the nucleotide derivative or its analog from being dried after the dotting, a substance having a high boiling point may be added in the aqueous solution in which the nucleotide derivative or its analog is dissolved or dispersed. The substance having a high boiling point is preferably a substance that can be dissolved in the aqueous solution in which the nucleotide derivative or its analog to be dotted is dissolved or dispersed, does not hinder hybridization with a sample such as a nucleic acid fragment sample (target nucleic acid fragment) which is an object of the detection, and has a not very high viscosity. Such substances include glycerin, ethylene glycol, dimethyl sulfoxide and a hydrophilic polymer having a lower molecular weight. Examples of the hydrophilic polymers include polyacrylamide, polyethylene glycol and sodium polyacrylate. Preferable molecular weight of this polymer is in the range from $10^3$ to $10^6$. As the substance having a high boiling point, it is more preferable to use glycerin or ethylene glycol, and particularly preferable to use glycerin. Preferable concentration of the substance having a high boiling point is in the range from 0.1 to 2% by volume, and particularly preferably 0.5 to 1% by volume in the aqueous solution of the nucleotide derivative or its analog.

Moreover, for the sake of the same purpose, it is also preferable to place the solid support after the dotting of a nucleotide derivative or its analog under the circumstances where the humidity is 90% or more and the temperature is in the range from 25 to 50 °C.

A post-treatment by ultraviolet ray, sodium borohydride or Schiff reagent may be carried out after the dotting of the nucleotide derivative or its analog having a reactive group. These post-treatments may be carried out by combining a plurality kinds of them, and the combination of heating treatment and the ultraviolet ray treatment is particularly preferable. These post-treatments are particularly effective in the case where the surface of the solid support is treated only by a polycationic compound. Incubation after the dotting is also preferable. After the incubation, removal of unreacted nucleotide derivatives or its analogs by washing is preferable.

A preferable fixed amount (numerical quantity) of nucleotide derivatives or its analogs with respect to the surface of the solid support is in the range from $10^2$ to $10^5$ per $cm^2$. A preferable amount of nucleotide derivatives or its analogs is in the range from 1 to $10^{-15}$ mol, and is several ng or less in weight. By dotting, the aqueous solution of nucleotide derivatives or its analogs is fixed in a dot shape to the surface of the solid support. The shape of the dot is nearly circular. No variation in the shape is important for the quantitative analysis of a gene expression and the analysis of single nucleotide polymorphism. A preferable distance between each dot is in the range from 0 to 1.5mm, and a particularly preferable distance is in the range from 100 to 300μm. As for the size of a dot, a preferable diameter of it is in the range from 50 to 300 μm. A preferable amount of the solution containing nucleotide derivatives or its analogs for dotting to the surface of the solid support is in the range from 100 pL to 1μL, and a particularly preferable amount is in the range from 1 to 100 nL.

FIG. 1 schematically shows a method for manufacturing an oligonucleotide fixed solid support which is a representative aspect of the present invention and a configuration of a representative oligonucleotide fixed solid support.

As a method for manufacturing a solid support having a porous substrate according to the present invention to which an oligonucleotide was fixed, in the case where a disulfone compound represented by the foregoing formula (2) is employed, four types of methods for manufacturing can be utilized depending on $X^1$ and $X^2$.

FIG. 1 shows (a) a method for manufacturing a solid support (C1) to which the oligonucleotide is fixed using a disulfone compound of the formula (2) of which both of $X^1$ and $X^2$ represent $-CHR^1-CR^2R^3Y$ (reactive precursor group), and (b) a method for manufacturing an oligonucleotide fixed solid support (C2) using the disulfone wherein $X^1$ represents $-CHR^1-CR^2R^3Y$ and $X^2$ represents $-CR^1=CR^2R^3$. Here, supposing that $X^1$ represents a group reacting in the first place with a reactive group (R) introduced on the surface of the solid support 1, a method for manufacturing the solid support using the disulfone where $X^1$ represent $-CR^1=CR^2R^3$ may also be useful. Hereinafter, description will be made supposing that "$X^1$" is a group reacting in the first place with a reactive group (R) introduced on the surface of the solid support 1.

The manufacturing methods (a) and (b) will be described below.

Step (1): $A-(CR^1R^2)_n-SO_2-L-SO_2-X^2$ group is introduced to the solid support by bringing a disulfone compound represented by the formula (1) into contact with the surface of the solid support 1 into which the reactive group (R) is introduced [solid support (A)], and substituting -Y portion of X1 with a reactive group (R).

Step (2): A reactive group (Z) is added by bringing an oligonucleotide 2 having a reactive group (Z) at one end into contact with $X^2$ of $-(CR^1R^2)_n-SO_2-L-SO_2-X^3$ group introduced in the Step (1), or -Y of the $X^2$ is substituted with the reactive group (Z) by bringing the oligonucleotide 2 into contact with Y.

A support of the present invention for fixing a probe molecule may be manufactured by the following method using $-CR^1=CR^2R^3$ as $X^1$, supposing that $X^1$ is a group reacting in the first place with the reactive group (R) introduced to the surface of the solid support 1.

Step (1): A $-R^3R^2C-R^1HC-SO_2-L-SO_2-X^2$ group is introduced to the surface of the solid support by bringing

a disulfone compound represented by the formula (I) into contact with the surface of the solid support (A) consisted of solid support having a porous substrate (solid support 1) to the surface of which the active group (R) is introduced, and adding the reactive group (R) to $-CR^1=CR^2R^3$ of $X^1$.

Step (2) : The reactive group (Z) is added by bringing an oligonucleotide having the reactive group (Z) at one end into contact with X2 of $-R^3R^2C-R_1HC-SO_2-L-SO_2-X_2$ group introduced in the Step (1), or -Y of the $X_2$ is substituted with the reactive group (Z) by bringing the oligonucleotide 2 into contact with Y.

The oligonucleotide having the reactive group (Z) at one end is a compound shown by the reference numeral 2 in FIG. 1. A crosslinker (Q) , though it is not essential, exists in general between the reactive group (Z) and phosphate ester group as a matter of convenience for preparation. A -phosphate group-NNNN,,,NN represents an oligonucleotide. $R^4$ represents a group determined by the reaction between the reactive group (R) and $X^1$, and $Z^1$ represents a group determined by the reaction between $X^2$ and the reactive group (Z), respectively.

When the oligonucleotide 2 having the reactive group (Z) is dotted to the surface of the solid support (B) having a porous substrate shown in FIG. 1, although the reaction between $X^2$ or -Y of $X^2$ and the reactive oligonucleotide fragment 2 is occurred, an unreacted $X^2$ to which the oligonucleotide 2 is not bound also exists on the surface of solid support (B) . In this case, there is a possibility that such $X^2$ reacts in a non-specific manner with a labeled nucleic acid fragment sample in a hybridization to be performed later resulting in a problem that non-specific binding may be measured. Therefore, it is preferable that the $X^2$ (i.e., a halogen atom of $X^2$, for example) has been previously subjected to a masking treatment. It is preferable that the masking treatment is performed by bringing an anionic compound having an amino group or a mercapto group into contact with the surface of the solid support (C) (or (C2)). Since the oligonucleotide 2 has a negative charge, the oligonucleotide 2 can be prevented from reacting with the unreacted $X^2$ by generating negative charge also on the surface of the solid support (C) . As for such an anionic compound, any one can be used if it reacts with a halogen atom of $X^2$ and has a negative charge ($COO^-$, $SO_3^-$, $OSO_3^-$, $PO_3^-$, or $PO_2^-$). Among them, an amino acid is preferable, and glycine or cysteine is particularly preferable. Further, taurine is also preferably used.

The shelf life of a solid support manufactured according to the present invention, in which a nucleotide derivative or its analog is fixed, is usually several weeks for a cDNA fixed solid support to which cDNA is fixed, and the shelf life of a solid support to which a synthesized oligonucleotide is fixed is further longer. A solid support of the present invention to which an oligonucleotide or its analog is fixed is utilized for monitoring the gene expressions, determining the base sequences, analyzing the mutations, analyzing the polymorphism or the like. The principle of the detection is based on the hybridization with the labeled sample nucleic acid fragment, which will be described later.

As a labeling method, an RI method and a non-RI method (fluorescence method, biotin method, chemiluminescence method or the like) are known, and in the present invention, use of the fluorescence method is preferable. As for a fluorescent substance utilized for a fluorescent labeling, although any can be used if it can bind to the basic portion of nucleic acid. For example, a cyanine dye (e.g., Cy3, Cy5 or the like of Cy Dye™ series, which is commercially available), rhodamine 6G reagent, N-acetoxy-N2-acetylaminofluorene (AAF) or AAIF (iodine derivative of AAF) can be used.

As a nucleic acid fragment sample, usually, a nucleic acid fragment sample such as a DNA fragment sample or a RNA fragment sample whose sequence and function is not known is used.

It is preferable that a nucleic acid fragment sample is isolated from the cell or tissue sample of eucaryote for the purpose of examining the gene expression. In the case where the sample is a genome, it is preferably isolated from any given tissue sample except for red blood cell. It is preferable that any given tissue except for red blood cell is peripheral blood lymphocyte, skin, hair, sperm or the like. In the case where the sample is an mRNA, it is preferable that it is extracted from the tissue sample in which the mRNA is expressed. It is preferable that a labeled cDNA is prepared from mRNA by incorporating a labeled dNTP ("dNTP" means a deoxyribonucleotide in which the base is adenine (A), cytosine (C), guanine (G) or thymine (T)) using reverse transcription reaction. As a dNTP, use of dCTP is preferable because of chemical stability. Although the amount of the mRNA required for one hybridization is different depending on the amount of liquid to be spotted, and the type of labeling material, it is several μg or less. It is desired that the nucleic acid fragment sample has been previously depolymerized in the case where a DNA fragment on the nucleotide derivative or its analog fixed solid support is an oligoDNA. In the case of a prokaryotic cell, since the selective extraction of an mRNA is difficult, it is preferable that the total RNA is labeled.

As for a nucleic acid fragment sample, for the purpose of examining the mutation and polymorphism of a gene, it is preferable to obtain it by performing PCR of the target region in a reaction system containing the labeled primer or the labeled dNTP.

It is preferable that hybridization is carried out by dotting an aqueous solution, which is previously pipetted into a 96 wells or 384 wells plastic plate, in which labeled nucleic acid fragment samples are dissolved or dispersed, to the solid support of the present invention to which nucleotide derivatives or its analogs are fixed. The preferable amount of the solution for dotting is in the range from 1 to 100 nL. The hybridization is preferably carried out in the temperature range from room temperature to 70°C, and for the period from 6 to 20 hours. After the completion of

hybridization, it is preferable that washing are performed using a mixed solution of a surfactant and a buffer solution to remove unreacted nucleic acid fragment samples. As a surfactant, it is preferable to use sodium dodecyl sulfate (SDS). As a buffer solution, citrate buffer solution, phosphate buffer solution, borate buffer solution, Tris buffer solution, Good' buffer solution or the like can be used. It is particularly preferable to use citrate buffer solution.

The hybridization using the solid support to which nucleotide derivatives or its analogs are fixed is characterized in that the amount of usage of the labeled nucleic acid fragment samples can be decreased to a very minute amount. Therefore, it is necessary to set the optimal conditions of the hybridization depending on the length of chain of the nucleotide derivatives or its analogs fixed to the solid support and the types of the labeled nucleic acid fragment samples. For the analysis of gene expression, it is preferable that a hybridization for a long time period is performed so as to be capable of sufficiently detecting even a lower expressing gene. For the detection of a single nucleotide polymorphism, it is preferable that a hybridization for a short period is performed. Moreover, it is also characterized in that comparison or quantitative determination of the expression amount are made possible using a single solid support to which DNA fragments are fixed by previously preparing two types of the nucleic acid fragment sample labeled by fluorescent substances different from each other and using them in a hybridization at the same time.

Furthermore, the present invention relates to a biological material chip, wherein A, which represents a residue of at least one protein or protein binding substance, is bound to a solid support having a porous substrate wherein the porous region has a fine pore diameter of about 2nm to about 1000nm, a porosity of about 10% to about 90% and a thickness of about 0.01µm to about 70µm, by covalent bond via a sulfonyl group as shown in the following formula (I)

$$\text{Solid phase support-L-}SO_2\text{-X-A} \qquad (I)$$

[In the above-described formula, L represents a linking group; X represents $-CR^1(R^2)-CR^3(R^4)-$; each of $R^1$, $R^2$, $R^3$ and $R^4$ represents, independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of a protein or a protein binding substance (except for nucleic acid)]

Representative examples of a protein or a protein binding substance to be fixed include an antibody or antibody fragment, a ligand, an antigen, an antigenic determinant such as a hapten or the like, a receptor, a ligand, avidins and a nucleic acid recognition protein, but are not limited thereto. Representative examples of a nucleic acid recognition protein include double strand recognition protein.

Avidins include avidin, streptavidin and these altered bodies that can form a stable complex with biotin. The phrase "can form a stable complex" means that a complex having a dissociation constant approximate to the dissociation constant of biotin-avidin complex ($10^{-15}$M) can be formed. The altered body means a body being modified or its fragment of avidin or streptavidin originally from the nature, or recombinants of these.

The nucleic acid recognition proteins include a double stranded DNA recognition substance, but are not limited thereto. The double stranded DNA recognition substances include a substance which recognizes a double stranded DNA and specifically binds to it. Examples of the double stranded DNA recognition substance include a DNA transcription factor, a mismatch repairing protein, a double stranded DNA recognition antibody, or a peptide nucleic acid. Furthermore, the double stranded DNA recognition substances include substances having Zinc Finger motif or Ring finger motif.

The DNA transcription factor is a substance that binds to the promoter region on gene and controls the transcription from DNA to mRNA (Takaaki, Tamura: Transcription Factor, published by Yodosha, Co., Ltd., 1995). Accordingly, it is known that the transcription factor specifically binds to a double stranded DNA of the specific sequence.

Among a large number of transcription factors, Zinc Finger Protein, that is, a transcription factor group having Zinc finger and Ring Finger motifs, shows a very high occurrence rate in eucaryote, and 1% of the genome seems to code for them. Plabo et al. have analyzed the tertiary structure of Zinc Finger motif and elucidated the mechanism of its binding to DNA (Science 252:809 (1991)). Further, Choo et al. have succeeded in preparing a Zinc Finger Protein group which binds to the specific sequence but which does not exist in the nature, by a gene recombinant method (Nature 372: 642 (1994), PNAS 91: 11163 (1994)). Furthermore, the Scripps Research Institute group has succeeded in preparing a novel Zinc Finger Protein group by Phage Display (PNAS 95: 2812 (1998) ; 96: 2758 (1999)). As described above, the DNA transcription factor group represented by Zinc Finger Protein originally has the nature of binding to a double stranded DNA, and according to the researches in recent years, it has been made possible to prepare a recombinant which recognizes a given DNA sequence. It is possible to efficiently capture a double stranded DNA on a support by fixing such proteins.

Besides these, the nucleic acid binding substances include a helix-loop-helix protein and a substance having an Ets domain.

The protein binding substances to be fixed (except for nucleic acid) include hapten, biotins (biotin, biocytin, desthiobiotin, oxybiotin or their derivatives that can form a stable complex with avidin). The phrase "can form a stable complex" means that a complex having a dissociation constant approximate to the dissociation constant of biotin-avidin complex ($10^{-15}$M) can be formed. Furthermore, peptides, sugars, hormones, pharmaceuticals, antibiotics and the like are listed.
A protein or a protein binding substance to be fixed to the solid support having a porous substrate forms a covalent bond via sulfonyl group with its amino group or mercapto group existing within it. Further, the covalent bond via sulfonyl group may be formed by introducing an amino group, an imino group, a hydrazine group, a carbomoyl group, a hydrazinocarbonyl group, a mercapto group or a carboxyimido group and the like into the protein.
A chip consisted of the solid support having a porous substrate to which a protein or a protein binding substance (e.g., antibody, avidins and nucleic acid recognition protein) is fixed, can fix the target substance on it, by bringing the chip into contact with a target substance (e.g., ligand, biotins and nucleic acid) that specifically react with the fixed protein or protein binding substance in the presence of an aqueous medium. It is desirable that a detectable label (e. g., fluorescene label, enzyme label or the like) is bound to the target substance of specific binding character to be fixed (e.g., ligand, biotins, and nucleic acid) so as to be capable of detecting its fixation from the exterior.
It is preferable that the solid support is a substrate having a flat and smooth surface. As a substance for the solid support, non-porous substances such as glass or plastic can be used.
The method for forming the porous region is as described above in the present specification. The porous substance and the organic polymer which constitutes the porous substrate are also as described above in the present specification.
In the present invention, a protein or protein binding substance A on the chip is bound to the solid support having a porous substrate by covalent bond via sulfonyl group as shown in the following formula (I):

$$\text{Solid phase support-L-}SO_2\text{-X-A} \qquad (I)$$

In the formula (I), L represents a linking group; X represents $-CR^1(R^2)-CR^3(R^4)-$; each of $R^1$, $R^2$, $R^3$ and $R^4$ represents independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of a protein or a protein binding substance (except for nucleic acid).
In the formula (I), L represents a bivalent or more linking group for binding $-SO_2$-X-A and the solid support. Examples of a -L- include any linking group selected from aliphatic, aromatic or heterocyclic compounds, hydro carbon chains that may be interrupted by a hetero atom, or combinations of them. Furthermore, L may be a singl bond.
In the formula (I), examples of an alkyl group having 1 to 6 carbon atoms include a methyl group, an ethyl group, a n-propyl group, a n-butyl group, and a n-hexyl group, and the methyl group is preferable. Examples of an aryl group having 6 to 20 carbon atoms include a phenyl group and a naphthyl group. It is preferable that each of $R^1$, $R^2$ and $R^3$ represents a hydrogen atom.
Examples of an aralkyl group having 7 to 26 carbon atoms in total containing an alkyl chain having 1 to 6 carbon atoms include the combination of the examples of an alkyl group having 1 to 6 carbon atoms and the examples of an aryl group having 6 to 20 carbon atoms.
Furthermore, the present invention relates to a method for detecting a target substance comprising the steps of:

bringing the chip in accordance with the present invention described above into contact with a specimen containing a target substance that specifically binds to a protein or a protein binding substance supported on the surface of the chip; and
detecting formation of reciprocal binding between the protein or protein binding substance and the target substance.

The type of "specimen" referred to in the present invention is not particularly limited, and includes, for example, a blood such as peripheral venous blood, white blood cell, serum, urine, stool, sperm, saliva, a cultured cell, a tissue cell such as a variety of cells of organs, and any other sample containing nucleic acid. As for the specimen, the sample such as the tissue cell as described above may be used as it is. However, preferably, nucleic acid, ligand or the like which has been released by destructing the cell in the specimen sample is used as a specimen. The destruction of the cell in the specimen sample can be performed according to the conventional manner. For example, it can be performed by adding a physical action such as shaking, supersonic treatment from the exterior. The nucleic acid also can be released from the cell using a nucleic acid extraction solution (e.g., solution containing a surfactant such as SDS, Triton-X, Tween-20 or the like, or saponin, EDTA, protease or the like, or the like) . In the case where the nucleic acid

is eluted using a nucleic acid extraction solution, the reaction can be promoted by incubation at the temperature of 37°C or higher.

Furthermore, the present invention relates to a method for manufacturing the chip according to the present invention comprising the step of contacting, the solid support having a porous substrate that contains a vinyl sulfonyl group or its reactive precursor group represented by the following formula (II) on the surface, with at least one protein or protein binding substance having a reactive group which forms a covalent bond by reacting with the vinyl sulfonyl group or its reactive precursor group.

$$-L-SO_2-X' \qquad (II)$$

[In the formula (II), L represents a linking group for binding $-SO_2-X'$ and the solid support; X' represents $-CR^1=CR^2(R^3)$ or $-CH(R^1)-CR^2(R^3)$ (Y) ; each of $R^1$, $R^2$ and $R^3$ represents independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and Y represents a group substituted by a nucleophilic reagent, or a group which is eliminated as "HY" by base]

In the formula (II), examples of an alkyl group having 1 to 6 carbon atoms include a methyl group, an ethyl group, a n-propyl group, a n-butyl group, and a n-hexyl group, and the methyl group is particularly preferable. Examples of an aryl group having 6 to 20 carbon atoms include a phenyl group and naphthyl group. It is preferable that each of $R^1$, $R^2$ and $R^3$ represents a hydrogen atom.

Examples of an aralkyl group having 7 to 26 carbon atoms in total containing an alkyl chain having 1 to 6 carbon atoms include the combination of the examples of an alkyl group having 1 to 6 carbon atoms and the examples of an aryl group having 6 to 20 carbon atoms.

In the formula (II), Y represents a group substituted by a nucleophilic reagent such as -OH, $-OR^0$, -SH, $NH_3$, $NH_2R^0$ ($R^0$ represents a group such as alkyl group or the like except for hydrogen atom) , or a group which is eliminated as "HY" by base. Examples thereof include a halogen atom, $-OSO_2R^{11}$, $-OCOR^{12}$, $-OSO_3M$, or a quaternary pyridinium group ($R^{11}$ represents an alkyl group having 1 to 6 carbon atoms, an aryl group having 6 to 20 carbon atoms, or an aralkyl group having 7 to 26 carbon atoms in total containing an alkyl chain having 1 to 6 carbon atoms; $R^{12}$ represents an alkyl group having 1 to 6 carbon atoms or halogenated alkyl group having 1 to 6 carbon atoms; M represents a hydrogen atom, an alkali metal atom, or an ammonium group).

An alkyl group of $R^{11}$, an aryl group of $R^{11}$ and an aralkyl group of $R^{11}$ may have a substituent. Examples of such a substituent include an atom or a group selected from the group consisted of a hydroxyl group, an alkoxy group having 1 to 6 carbon atoms, an alkenyl group having 1 to 6 carbon atoms, a carbamoyl group having 2 to 7 carbon atoms, an alkyl group having 1 to 6 carbon atoms, an aralkyl group having 7 to 16 carbon atoms, an aryl group having 6 to 20 carbon atoms, an sulfamoyl group (or its sodium salt, potassium salt or the like) , a sulfo group (or its sodium salt, potassium salt or the like) , a carboxylic acid group (or its sodium salt, potassium salt or the like), a halogen atom, an alkenylene group having 1 to 6 carbon atoms, an arylene group having 6 to 20 carbon atoms, sulfonyl group and their combinations.

In the formula (II), L represents a bivalent or more linking group for linking $-SO_2-X'$ group and the solid support. Examples of a -L- include any linking group selected from aliphatic, aromatic or heterocyclic compound and a hydrocarbon chain that may be interrupted by a hetero atom, and a linking group selected from their combinations. Further L may be a single bond.

Preferable examples of the above-described "-X' " group are shown thereinafter:

(X1) $-CH=CH_2$

(X2) $-CH_2CH_2-Br$

(X3) $-CH_2CH_2-Cl$

(X4) $-CH_2CH_2-OSO_2CH_3$

(X5) $-CH_2CH_2-OSO_2C_6H_5$

(X6) $-CH_2CH_2-OSO_2C_6H_4-CH_3$

(X7)

$-CH_2CH_2\text{-}OSO_3Na$

(X8)

$-CH_2CH_2\text{-}OSO_3K$

(X9)

$-CH_2CH_2\text{-}OCOCH_3$

(X10)

$-CH_2CH_2\text{-}OCOCF_3$

(X11)

$-CH_2CH_2\text{-}OCOCHCl_2$

(X12)

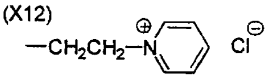

(X13)

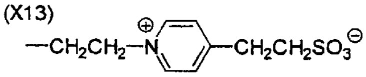

(X14)

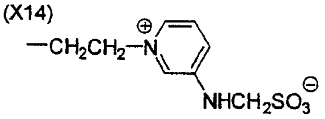

In the above-described concrete examples, it is preferable that "-X'" represents (X1), (X2), (X3), (X4), (X7), (X8) , (X13) or (X14), and more preferable that "-X'" represents (X1) or (X2). And it is particularly preferable that "-X' " represents a vinyl group represented by (X1).

Since a covalent bond via a sulfonyl group utilized in the present invention has a high resistance to hydrolysis, it can be readily stored in a stable state, and can rapidly react with the protein in which an amino group or a mercapto group has been previously provided to form a stable covalent bond.

Usually, it is not necessary to introduce another reactive group, since the protein has an amino group or a mercapto group. However, since the tertiary structure of the protein largely participates in its function, in the case where the activity of the protein is lowered, it is preferable to introduce a reactive group at the specific position having no influence on the activity.

Dotting of the protein or protein binding substance is carried out by dotting an aqueous solution to the surface of the reactive solid support. Concretely, it is preferable to carry out the dotting along the following steps of: dissolving or dispersing the protein or protein binding substance into the aqueous medium to prepare an aqueous solution; pipetting the aqueous solution into a 96 wells or 384 wells plastic plate; dropping the aqueous solution thus pipetted to the surface of the solid support using a spotter device or the like. As is described above, although the spotter device can be used for dropping the protein or protein binding substance, there is a possibility of lowering the activity of the protein or protein binding substance depending on the property of a pin-head. In this case, it may be more preferable to use an ink jet device or the like depending on a case.

In order to prevent the protein or protein binding substance from being dried after the dotting of it, a substance having a high boiling point may be added in the aqueous solution. As a substance having a high boiling point, the substance that can be dissolved in the aqueous solution in which protein or protein binding substance to be dotted is dissolved or dispersed, does not hinder reaction with a sample which is an object of the detection, and has a not very high viscosity is preferably used. Examples of such substances include glycerin, ethylene glycol, dimethyl sulfoxide and a hydrophilic polymer having a lower molecular weight. The hydrophilic polymers include polyacrylamide, polyethylene glycol and sodium polyacrylate. Preferable molecular weight of this polymer is in the range from $10^3$ to $10^6$. As the substance having a high boiling point, it is more preferable to use glycerin or ethylene glycol, and particularly preferable to use glycerin. Preferable concentration of the substance having a high boiling point can be adjusted depending on the activity of the protein after the dotting.

Moreover, for the sake of the same purpose, it is also preferable to place the solid support after the dotting of the protein or protein binding substance under the circumstances where the humidity is 90% or more and the temperature is in the range from 25 to 40°C.

A preferable fixed amount (numerical quantity) of the protein or protein binding substance with respect to the surface of the solid support is in the range from 1 to $10^5$ per $cm^2$. By the dotting, the aqueous solution of the protein or protein binding substance is fixed in a dot shape to the surface of the solid support. The shape of the dot is nearly circular. The distance between the respective dots, the size of the dot and the volume of the aqueous solution when it is dotted vary depending on its intended use.

FIG. 2 schematically shows a configuration of a protein chip, which is the representative aspect of the present invention.

When the protein A having the reactive group (Z) is dotted to the surface of the porous solid support (P1) shown in FIG. 2, the reaction between X and the protein occurs. However, an unreacted X to which the protein is not bound also exists on the surface of the solid support (P1). In this case, there is a possibility that such X reacts in a non-specific manner with a labeled ligand sample and the like in a reaction to be performed later, resulting in a problem that non-specific binding may be measured. Therefore, it is preferable that the X has been previously subjected to a blocking treatment. It is preferable that the blocking treatment is performed by bringing a compound having an amino group or a mercapto group into contact with the surface of the solid support (P2). In order to prevent the non-specifical binding of a ligand to be reacted, it is preferable to perform the blocking treatment using a protein blocking agent, specifically, BSA, casein, gelatin or the like. As the result of the blocking, BSA or the like exists on the surface of the solid support (P2) which has not been dotted, thereby preventing the binding of the ligand. Moreover, in the case where the nucleic acid is to be reacted, the blocking treatment can be carried out by contacting an anionic compound having an amino group or a mercapto group other than the above-described protein blocking agent. In the case where the substance with which the protein is reacted is a nucleic acid, since the nucleic acid has a negative charge, it can prevent the nucleic acid from reacting with an unreacted X by generating the negative charge also on the surface of the solid support (P2). As for such an anionic compound, any compound can be used if it reacts with X and has a negative charge ($COO^-$, $SO_3^-$, $OSO_3^-$, $PO_3^-$, or $PO_2^-$). Among them, an amino acid is preferable, and glycine or cysteine is particularly preferable. Further, taurine is also preferably used.

A protein chip which is a representative aspect of the present invention is utilized for the analysis of protein interactions, the analysis of the protein expression and the drug development search. Furthermore, in the case where the protein is a nucleic acid binding protein, it can be utilized for the mutation analysis and the nucleotide polymorphism analysis depending on its recognition nucleic acid sequence.

The principle of the detection is based on the reaction with the labeled ligand or nucleic acid. As labeling methods, although a RI method and a non-RI method (fluorescence method, biotin method, chemiluminescence method or the like) are known, it is not particularly limited. For example, in the case of the fluorescence method, as a fluorescent substance utilized for a fluorescence labeling, any can be used if it can bind to the basic portion of nucleic acid or protein amino acid residue. For example, cyanine dye (e.g., Cy3, Cy5 or the like of Cy Dye™ series, which is commercially available) , rhodamine 6G reagent, N-acetoxy-N2-acetylaminofluorene (AAF) or AAIF (iodine derivative of AAF) can be used.

In the case of a protein chip in which a nucleic acid recognition protein is fixed, it is preferable that the target nucleic acid in the specimen is directly detected without amplifying it by a PCR method or the like. However, it may be detected after it has been previously amplified. The target nucleic acid or its amplified body can be easily detected by previously labeling it. In order to label the nucleic acid, a method of using an enzyme (Reverse Transcriptase, DNA polymerase, RNA polymerase, Terminal deoxy trasferase or the like) is often used. Further, the labeling substance may be directly bound by chemical reaction. Such labeling method has been described in some books as the known technology (Shintaro Nomura: De-isotope Experiment Protocol 1, published by Shujun Sha, Co., Ltd., 1994; Shintaro Nomura: De-isotope Experiment Protocol 2, published by Shujun Sha, Co., Ltd., 1998; Masaaki Muramatsu: DNA Microarray and Advanced PCR Method Labeling Material, published by Shujun Sha, Co., Ltd., 2000). It is preferable that the labeling material is a material capable of making a detectable signal. In the case where the labeling material is a material having an amplifying ability of signal such as an enzyme and a catalyst, the detection sensitivity of DNA is largely enhanced.

However, since the labeling operation described above is generally troublesome, a method of measuring the nucleic acid in the specimen without previous labeling of the nucleic acid can be used as a more preferable method of detection. For the purpose of it, for example, a DNA intercalating agent which recognizes a double stranded DNA, that is, what is called a DNA intercalator can be used. By the use of a DNA intercalator, not only the operation of the detection is made easier, but also the sensitivity of the detection is enhanced. For example, in the case where a DNA of 1000 bp is to be detected, although a labeling method can introduce several labeling materials at most, in the case where the intercalator is used, 100 or more labeling materials can be introduced.

The DNA intercalator may be a material which can form a detectable signal in itself, or the signal formation material may be bound to the side chain of intercalator, or bound to the intercalator via a specific binding pair such as a biotin-avidin, an antigen-antibody or a hapten-antibody. It is preferable that the detectable signal used in the present invention is the signal detectable by a fluorescene detection, a luminescence detection, a chemiluminescence detection, a bioluminescence detection, an electrochemiluminescence detection, a radiation detection, an electrochemical detection or a colorimetric detection, but it is not limited to them.

In the case where a ligand is a target, there can be used a substance obtained by reacting a succinimide body of a cyanine dye (e.g., Cy3, Cy5 or the like of Cy Dye™ series, which is commercially available), rhodamine 6G reagent, N-acetoxy-$N_2$-acetylaminofluorene (AAF) or AAIF (iodine derivative of AAF) with an amino group existing

inside.

The present invention will be more concretely described below by the following Examples, but the present invention is not limited by these Examples.

## Examples

### Example 1: Preparation of DNA fragment fixed slide, and measurement of fixed amount of DNA fragment

The preparation and its performance of a reactive solid support having a porous substrate, and the comparison with a reactive solid support not having a porous substrate as a comparative control are shown below.

(1) Preparation of porous solid support (A)

15g of 15% suspension of a colloidal silica (Snowtechs PS-S[Nissan Chemical Industries Co., Ltd.]/average particle diameter about 10 nm), 13g of methanol, 5g of water and 1g of TMOS were mixed and stirred for 10 minutes, and the mixture was stand left for 1 hour. The solution was filtered by 0.22 micron filter. Glass slides were immersed in the obtained solution, and were dried at room temperature for 2 hours. Then, the slides glasses were heated at 70°C for 1 hour, and at 100°C for 2 hours. Next, each glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using slide washer) with 200 ml of ultrapure water. While exchanging the ultrapure water, washings were repeated more two times under the above-described washing conditions. After the completion of washings, and after it had been dried for 10 minutes by the drier at 45°C, it was put into the oven set at 110°C was thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20 second washing was repeated three times with ultrapure water. The drying was performed for 30 minutes by the drier set at 25°C to obtain a porous solid support (A).

The deflection of the surface of the porous solid support (A) was measured so as to obtain an index of refraction of membrane of about 1.3. Since the index of refraction of porous membrane is a volume average of solid phase and fine pore space, estimate of porosity can be obtained. From the above measurement, it was estimated that the above membrane had a porosity of about 30-40%. Moreover, the thickness of the membrane was determined to be about 6500 angstrom. The fin pore diameter is estimated by assuming three particles of the same size within a triangle layer. In this configuration, the fine pore diameter is about 0.15 times greater than the diameter of the particle. It is estimated that the above 10nm particle has a finest pore diameter of about 2nm. The above measurements were carried out in the same way as in International Publication WO00/61282.

(2) Dotting of DNA fragment and measurement of fluorescence intensity

The aqueous solution ($1 \times 10^{-6}$M, 1μL) consisted of dispersion of the DNA fragment (3'-TCCTCCATGTCCGGGGAGGATCTGACACTTCAAGGTCTAG-5'), of which 3' end and 5' end were modified with an amino group and a fluorescence labeling reagent (Fluoro Link Cy5-dCTP, made by Amersham Pharmacia Biotech, Co., Ltd.) respectively, in 0.1M carbonate buffer solution (pH 9.3) , was dotted on the slide (A) obtained in the above-described (1). Immediately, the solid support after the dotting was left for 1 hour at the temperature of 25°C and humidity of 90%. The solid support was washed with the mixed solution of 0.1 % by weight SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine aqueous solution (pH 10) for 1 hour and 30 minutes, washed with distilled water, and dried at room temperature to obtain a solid support (B) to which DNA fragments were fixed. The fluorescence intensity of the surface of this solid support (B) measured by a fluorescence scanning device was 25,690.

(3) Preparation of a comparative control slide, and its performances

The glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using the slide washer) with 200 ml of ultrapure water. While exchanging the ultrapure water, washings were repeated more two times under the above-described washing conditions. After the completion of washings, it was dried for 10 minutes by the drier at 45°C, then put into the oven set at 110°C and thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20

second washing was repeated three times with ultrapure water. The drying was performed for 30 minutes by the drier set at 25°C. Evaluation similar to the above-described (2) was performed, and 1900 of the fluorescence intensity was obtained.

From the above results, it is understood that, according to the fixation method of the present invention, DNA fragments are efficiently fixed in a high density on the slide glass.

Example 2: Detection of sample DNA fragment

(1) Preparation of DNA fragment fixed porous solid support

The aqueous solution (1 x $10^{-6}$M, 1 μL) consisted of dispersion of 40 mer of the DNA fragment (3'-TCCTCCATGTCCGGGGAGGATCTGACACTTCAAGGTCTAG-5'), of which 3' end was modified with an amino group , in 0.1M carbonate buffer solution (pH 9.3), was dotted on the porous solid support (A) obtained in the above-described (1) of Example 1. Immediately, the solid support after the dotting was left for 1 hour at the temperature of 25°C and humidity of 90%. The solid support was washed with the mixed solution of 0.1 % by weight SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine aqueous solution (pH 10) for 1 hour and 30 minutes, washed with distilled water, dried at room temperature to obtain a solid support (C) to which DNA fragments were fixed.

(2) Detection of sample DNA fragment

The aqueous solution consisted of dispersion of 22 mer of the sample oligonucleotide (CTAGTCTGTGAAGTTCCAGATC-5'), of which 5' end was bound with Cy5, in the solution for hybridization (mixed solution of 4 x SSC and 10% by weight of SDS) (20μL) was dotted on the solid support (C) obtained in the above-described (1). Then, the surface was protected by a cover glass for microscopy, and incubated at 60°C for 20 hours within a moisture chamber. After the incubation, it was washed with the mixed solution of 0.1% by weight SDS and 2 x SSC, the mixed solution of 0.1% by weight SDS and 0.2 x SSC, and 0.2 x SSC aqueous solution in turn, centrifuged at 600 rpm for 20 seconds, and dried at room temperature. Fluorescence intensity of the surface of the glass slide measured by a fluorescence scanning device was 19600.

On the other hand, when the detection of the sample DNA fragment was performed using the comparative control slide, the fluorescence intensity was 1500.

It is understood that a sample DNA fragment having the complementarity to the fixed DNA fragment can be detected in a high sensitivity by employing the DNA fragment fixed porous solid support prepared according to a fixation method of the present invention.

Example 3: Detection of ligand by antibody fixed slide

(1) Preparation of porous solid support (A) to which vinyl sulfonyl group has been introduced

15g of 15% suspension of a colloidal silica (Snowtechs PS-S[Nissan Chemical Industries Co., Ltd.]/average particle diameter about 10 nm), 13g of methanol, 5g of water and 1g of TMOS were mixed and stirred for 10 minutes, and the mixture was stand left for 1 hour. The solution was filtered by 0.22 micron filter. Glass slides were immersed in the obtained solution, and were dried at room temperature for 2 hours. Then, the slides glasses were heated at 70°C for 1 hour, and at 100°C for 2 hours. Next, each glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using slide washer) with 200 ml of ultrapure water. While exchanging the ultrapure water, washings were repeated more two times under the above-described washing conditions. After the completion of washings, and after it had been dried for 10 minutes by the drier at 45°C, it was put into the oven set at 110°C was thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20 second washing was repeated three times with ultrapure water. The drying was performed for 30 minutes by the drier set at 25°C to obtain a porous solid support (A).

The deflection of the surface of the porous solid support (A) was measured so as to obtain an index of refraction of membrane of about 1.3. Since the index of refraction of porous membrane is a volume average of solid phase and fine pore space, estimate of porosity can be obtained. From the above measurement, it was estimated that the above membrane had a porosity of about 30-40%. Moreover, the thickness of the membrane was determined to be about 6500 angstrom. The fin pore diameter is estimated by assuming three particles of the same size within a triangle layer.

In this configuration, the fine pore diameter is about 0.15 times greater than the diameter of the particle. It is estimated that the above 10nm particle has a finest pore diameter of about 2nm. The above measurements were carried out in the same way as in International Publication WO00/61282.

(2) Fixation of antibody

Goat Anti-Human IgG (Jackson ImmunoResearch) was diluted with PBS (100, 20, 4, 0.8, 0.16 ng/μL, 1μL), and dotted on the solid support (A) prepared in the above-described (1). Immediately, the solid support after the dotting was left at 25°C for 3 hours in a saturated common salt chamber. Then, the blocking treatment was performed by immersing it in 1% BSA/0.05% Tween 20-PBS (PBS-T) for 1 hour to obtain an antibody slide (B) .

(3) Reaction with ligand and detection of it

HybriWell (Grace Bio-Labs) was intimately contacted to' the antibody slide (B) prepared in the above-described (2). Human IgG-Cy5 (Jackson ImmunoResearch) was diluted with 1% BSA/PBS-T into 2μg/ml. After 100μL of the diluted solution was added within HybriWell, it was incubated at 25 °C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 26.3 at the position where the antibody was spotted with 100 ng/μL, indicating a large increase from a background fluorescence intensity. Therefore, it is understood that by employing the antibody fixed solid support of the present invention consisted of the porous solid support to which antibodies have been bound via sulfonyl group, a ligand having reactivity with the antibody fixed on the antibody fixation solid support can be efficiently detected.

Example 4: Detection of ligand by antibody fixed slide

(1) Fixation of antibody

Rabbit Anti Streptavidin (Polysienece) was diluted with PBS (100 ng/μL), and each 10μL of it was pipetted into 384 wells plate. It was dotted on the solid support (A) prepared in the above-described Example 3 (1) using an arrayer made by Cartecyan (PixSys 5500). Immediately, the solid support after the dotting was left at 25°C for 3 hours in a saturated common salt chamber, then the blocking treatment was performed by immersing it in 1/4 x Block Ace (Dainippon Pharmaceuticals)/0.05% Tween 20-PBS (PBS-T) for 1 hour to obtain an antibody slide (C).

(2) Labeling of ligand

1 mg of Streptavidin (Wako Junyaku) was dissolved in 400 μl of 0.1 M $NaHCO_3$ (pH 8.0). It was transferred to a Cy3-monofunctional tube (Amersham Pharmacia Biotech) and incubated at room temperature for 30 minutes. 100 μl of 1 M Tris/HCl (pH 8.0) was added to it, and further incubated at room temperature for 30 minutes to stop the reaction. The reacted solution was purified by NAP-5 column (Amersham Pharmacia Biotech), and Cy3-labeled Streptavidin was obtained.

(3) Reaction with ligand and detection of it

SecureSeal Hybridization Chamber (SA 500, Grace Bio-Labs) was intimately contacted to the antibody slide (C) prepared in the above-described (1). Cy3-labeled Streptavidin obtained in the above-described (2) was diluted with 1/4 x Block Ace/PBS-T into 2 μg/ml. After 500 μL of it was added within SA 500, it was incubated at 25°C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 21,300, indicating a large increase from the background fluorescence intensity. Therefore, it is understood that by employing the antibody fixed solid support of the present invention consisted of the porous solid support to which antibodies have been bound via sulfonyl group, a ligand having reactivity with the antibody fixed on the antibody fixed solid support can be efficiently detected also in a microarray system.

Industrial Applicability

By utilizing a fixation method of the present invention, a probe of a nucleotide derivative or its analog nucleotide such as an oligonucleotide, a polynucleotide, or a peptide nucleic acid can be fixed in high density with high stability on the surface of a solid support having porosity. Therefore, the solid support to which the nucleotide derivative or its

analog is fixed by the fixation method of the present invention forms the solid support with high stability, in which release of the probe resulted from hydrolysis hardly occurs. Particularly, in the case where an amino group or the like is introduced using a silane coupling agent to surface of the solid support, the probe can be firmly fixed to the solid support, since both of the binding of the amino group or the like to the surface of the solid support and the binding of the probe are formed by the covalent bond. A detection tool having a high detection limit which is capable of being utilized for gene analysis or the like can be obtained by this stable fixation of the probe.

As one example of it, a nucleic acid fragment sample having complementarity to the probe fixed on an oligonucleotide fixed solid support can be detected in a high sensitivity by performing the hybridization with the sample nucleic acid fragment using an oligonucleotide fixed solid support prepared according to the present invention. Moreover, a non-specific absorption of the sample nucleic acid fragment can be prevented by performing the treatment of the solid support surface with an anionic compound such as glycine after the probe sample such as the oligonucleotide is dotted on the surface of the reactive solid support, thereby exerting a larger effect on the detection with a high sensibility of a nucleic acid fragment sample having the complementarity.

Furthermore, according to the present invention, there can be provided a biological material chip wherein at least one member of specific binding partners is bound and fixed on a reactive solid support which is capable of rapidly and stably binding and fixing

## Claims

1. A reactive solid support having a porous substrate wherein the porous region has a fine pore diameter of about 2nm to about 1000nm, a porosity of about 10% to about 90% and a thickness of about 0.01μm to about 70μm, to

the surface of which a group of vinyl sulfonyl groups or their reactive precursor groups are fixed by covalent bond via a linking group, respectively.

**2.** The reactive solid support as claimed in claim 1, wherein the porous substrate is composed of an organic polymer.

**3.** The reactive solid support as claimed in claim 1, wherein the porous substrate is composed of an inorganic substrate.

**4.** The reactive solid support as claimed in claim 1, wherein the porous substrate comprises silicon, alumina or titanium.

**5.** The reactive solid support as claimed in claim 1, wherein a linked body of the vinylsulfonyl group or its reactive precursor group and the linking group is represented by the following formula:

$$-L-SO_2-X$$

in the above-described formula, X represents $-CR^1=CR^2R^3$ or $-CHR^1-CR^2R^3Y$, each of $R^1$, $R^2$ and $R^3$ represents independently from each other an atom or a group selected from the group consisted of a hydrogen atom, an alkyl group having 1 to 6 carbon atoms, an aryl group having 6 to 20 carbon atoms, and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and a quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group.

**6.** The reactive solid support as claimed in claim 5, wherein X represents a vinyl group represented by $-CH=CH_2$.

**7.** The reactive solid support as claimed in claim 5, wherein L represents a linking group containing an atom of a bivalence or more except for carbon atom.

**8.** The reactive solid support as claimed in claim 5, wherein L represents a linking group having a linking portion selected from the group consisted of -NH-, -S- and -O-.

**9.** The reactive solid support as claimed in claim 5, wherein L represents a linking group represented by $-(L^1)_n-NH-(CR^1R^2)_2-$ or $-(L_1)_n-S-(CR^1R_2)_2-$ wherein $R^1$ and $R^2$ represents the same meanings as described above, $L^1$ represents a linking group, and n represents either 0 or 1.

**10.** The reactive solid support as claimed in claim 5, wherein L represents a linking group represented by $-(L^1)_n-NHCH_2CH_2-$ wherein $L^1$ represents a linking group, and n represents either 0 or 1.

**11.** The reactive solid support as claimed in claim 9, wherein $L^1$ represents a linking group containing a group represented by -OSi-, and n represents 1.

**12.** The reactive solid support as claimed in claim 1, wherein said solid support is a substrate in a sheet shape selected from the group consisted of a glass substrate, a resin substrate, a glass substrate or a resin substrate surface-treated with a silane coupling agent and a glass substrate or a resin substrate having a covering layer on its surface.

**13.** The reactive solid support as claimed in claim 12, wherein said solid support is a substrate in a sheet shape selected from the group consisted of a silicate glass substrate, a silicate glass substrate surface-treated with a silane coupling agent and a silicate glass substrate covered by an organic covering layer.

**14.** A method of manufacturing the reactive solid support as claimed in claim 5, wherein a disulfone compound represented by the following formula is brought into contact with a reactive solid support to the surface of which a reactive group is introduced:

$$X^1\text{-}SO_2\text{-}L^2\text{-}SO_2\text{-}X^2$$

in the above-described formula, each of $X^1$ and $X^2$ represents independently from each other $-CR^1=CR^2R^3$ or $-CHR^1-CR^2R^3Y$, each of $R^1$, $R^2$ and $R^3$ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and a quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and $L^2$ represents a linking group.

**15.** The method of manufacturing a reactive solid support as claimed in claim 14, in which the reactive group introduced to the surface of said solid support is an amino group, a mercapto group or a hydroxyl group.

**16.** A manufacturing method of a solid support comprising a nucleotide derivative or its analog bound to the surface of the support via a linking group having a sulfonyl group, wherein a surface of a reactive solid support having a porous substrate on which each of a group of vinylsulfonyl group or its reactive precursor group is fixed by covalent bond, is contacted with a nucleotide derivative or its analog having a reactive group which is capable of reacting with said reactive group to form a covalent bond.

**17.** The manufacturing method as claimed in claim 16, wherein said nucleotide derivative or its analog is selected from the group consisted of an oligonucleotide, a polynucleotide and a peptide nucleic acid.

**18.** The manufacturing method as claimed in claim 16, wherein a reactive solid support where a linked body of a vinylsulfonyl group or its reactive precursor group represented by the following formula and a linking group is bound to and fixed on, is used as a reactive solid support having a porous substrate:

$$-L\text{-}SO_2\text{-}X$$

in the above-described formula, X represents $-CR^1=CR^2R^3$ or $-CHR^1-CR^2R^3Y$, each of $R^1$, $R^2$ and $R^3$ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, $-OSO_2R^{11}$, $-OCOR^{12}$, $-OSO_3M$ and a quaternary pyridinium group; $R^{11}$ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; $R^{12}$ represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group or a single bond.

**19.** The manufacturing method as claimed in claim 18, wherein X represents a reactive group represented by $-CR^1=CR^2R^3$ wherein each of $R^1$, $R^2$ and $R^3$ represents the same meanings as described above.

**20.** A solid support having a porous substrate, in which a nucleotide derivative or its analog obtained by a manufacturing method claimed in claim 16 is bound and fixed to surface of the solid support.

**21.** A method of binding and fixing a complementary oligonucleotide or polynucleotide, wherein the solid support having a porous substrate to which the nucleotide derivative or its analog is bound as claimed in claim 20, is contacted with an oligonucleotide or a polynucleotide having complementarity to said fixed nucleotide derivative or its analog in the presence of an aqueous medium.

**22.** The method as claimed in claim 21, wherein a detectable label is bound to said complementary oligonucleotide

or polynucleotide.

**23.** A solid support to which a oligonucleotide or a polynucleotide having complementarity is bound and fixed wherein, to the solid support having a porous substrate to which the nucleotide derivative or its analog is bound, as claimed in claim 20, a oligonucleotide or a polynucleotide having complementarity to said fixed nucleotide derivative or its analog is bound in a complementary manner.

**24.** The solid support as claimed in claim 23, wherein a detectable label is bound to said oligonucleotide or polynucleotide having the complementarity.

**25.** A method of identifying or screening a gene, wherein the solid support having a porous substrate, to the surface of which the nucleotide derivative or its analog is bound and fixed as claimed in claim 20, or the solid support to which the complementary oligonucleotide or polynucleotide is bound and fixed as claimed in claim 23, is utilized.

**26.** A biological material chip, wherein A, which represents a residue of at least one protein or protein binding substance, is bound to a solid support having a porous substrate wherein the porous region has a fine pore diameter of about 2nm to about 1000nm, a porosity of about 10% to about 90% and a thickness of about 0.01µm to about 70µm, by covalent bond via a sulfonyl group as shown in the following formula (I):

$$\text{Solid support-L-SO}_2\text{-X-A} \qquad \text{(I)}$$

in the formula (I), L represents a linking group; X represents $-CR^1(R^2)-CR^3(R^4)-$; each of $R^1$, $R^2$, $R^3$ and $R^4$ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and A represents a residue of a protein or protein binding substance except for nucleic acid.

**27.** The chip as claimed in claim 26, wherein said protein or protein binding substance bounded to the surface is an antibody, an antibody fragment, a ligand, an antigen, a hapten or a receptor.

**28.** The chip as claimed in claim 27, wherein said protein or protein binding substance bound to the surface is avidins.

**29.** The chip as claimed in claim 28, in which avidins are an avidin, a streptoavidin or altered bodies thereof which are capable of forming a stable complex with a biotin.

**30.** The chip as claimed in claim 26, wherein said protein bound to the surface is a nucleic acid recognition protein.

**31.** The chip as claimed in claim 30, in which said nucleic acid recognition protein is a double stranded DNA recognition protein.

**32.** The chip as claimed in claim 31, wherein said double stranded DNA recognition protein is a double stranded DNA recognition antibody.

**33.** The chip as claimed in claim 31, wherein said double stranded DNA recognition protein is a DNA transcription factor.

**34.** The chip as claimed in claim 31, wherein said double stranded DNA recognition protein is a protein having a Zinc finger motif or a Ring finger motif.

**35.** The chip as claimed in claim 26, wherein the porous substrate is composed of an organic polymer.

**36.** The chip as claimed in claim 26, wherein the porous substrate is particle composed of an inorganic substance.

**37.** The chip as claimed in claim 26, wherein the porous substrate comprises silicon, alumina or titanium.

**38.** The chip as claimed in claim 26, wherein said solid support is a glass, a plastic, an electrode surface or a sensor chip surface.

**39.** A method of detecting a target substance, comprising the steps of:

contacting the chip claimed in claim 26 with a sample containing a target substance which specifically binds to a protein or a protein binding substance except for nucleic acid supported on the surface of said chip; and detecting formation of reciprocal binding between said protein or protein binding substance and said target substance.

**40.** The method of detecting a target substance as claimed in claim 39, wherein said target substance is labeled with at least one component capable of generating a detectable signal.

**41.** The method of detecting a target substance as claimed in claim 39, comprising a step of performing blocking processing of the chip with an aqueous solution of an amino acid, a peptide or a protein.

**42.** The method of manufacturing the chip claimed in claim 26 comprising a step in which a solid support having a porous substrate which contains a vinylsulfonyl group or its reactive precursor group represented by the following formula (II) on its surface is contacted with at least one protein or protein binding substance having a reactive group which forms a covalent bond by reacting with said vinylsulfonyl group or its reactive precursor group:

$$-L-SO_2-X' \qquad (II)$$

in the above-described formula (II), L represents a linking group which binds $-L-SO_2-X'$ to a solid support; X' represents $-CR^1=CR^2(R^3)$ or $-CH(R^1)-CR^2(R^3)$ (Y) ; each of $R^1$, $R^2$ and $R^3$ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and Y represents a group which is substituted by a neucleophilic reagent or a group which is eliminated as a "HY" by a base

**43.** The method of manufacturing a chip as claimed in claim 42, wherein said protein or protein binding substance bound to the surface is an antibody, an antibody fragment, a ligand, an antigen, a hapten or a receptor.

**44.** The method of manufacturing a chip as claimed in claim 42, wherein said protein or protein binding substance bound to the surface is avidins.

**45.** The method of manufacturing a chip as claimed in claim 44, wherein avidins are an avidin, a streptoavidin or altered bodies thereof which are capable of forming a stable complex with a biotin.

**46.** The method of manufacturing a chip as claimed in claim 42, wherein said protein bound to the surface is a nucleic acid recognition protein.

**47.** The method of manufacturing a chip as claimed in claim 46, wherein said nucleic acid recognition protein is a double stranded DNA recognition protein.

**48.** The method of manufacturing a chip as claimed in claim 47, wherein said double stranded DNA recognition protein is a double stranded DNA recognition antibody.

**49.** The method of manufacturing a chip as claimed in claim 47, wherein said double stranded DNA recognition protein is a DNA transcription factor.

**50.** The method of manufacturing a chip as claimed in claim 47, wherein said double stranded DNA recognition protein is a protein having a Zinc finger motif or a Ring finger motif.

**51.** The method of manufacturing a chip as claimed in claim 42, wherein said porous substrate is composed of an organic polymer.

**52.** The method of manufacturing a chip as claimed in claim 42, wherein said porous substrate is particle composed of an inorganic substance.

**53.** The method of manufacturing a chip as claimed in claim 42, wherein said porous substrate comprises silicon, alumina or titanium.

**54.** The method of manufacturing a chip as claimed in claim 42, in which a solid support is a glass, a plastic, an electrode surface or a sensor chip surface.

**55.** The method of manufacturing a chip as claimed in claim 42, comprising the steps of:

fixing at least one protein or protein binding substance to a solid support having a porous substrate by contacting said protein or protein binding substance to said solid support; and
performing blocking process of a free vinylsulfonyl group or its reactive precursor group located on the surface of said solid support with an amino acid, a peptide or a protein aqueous solution.

Fig.1

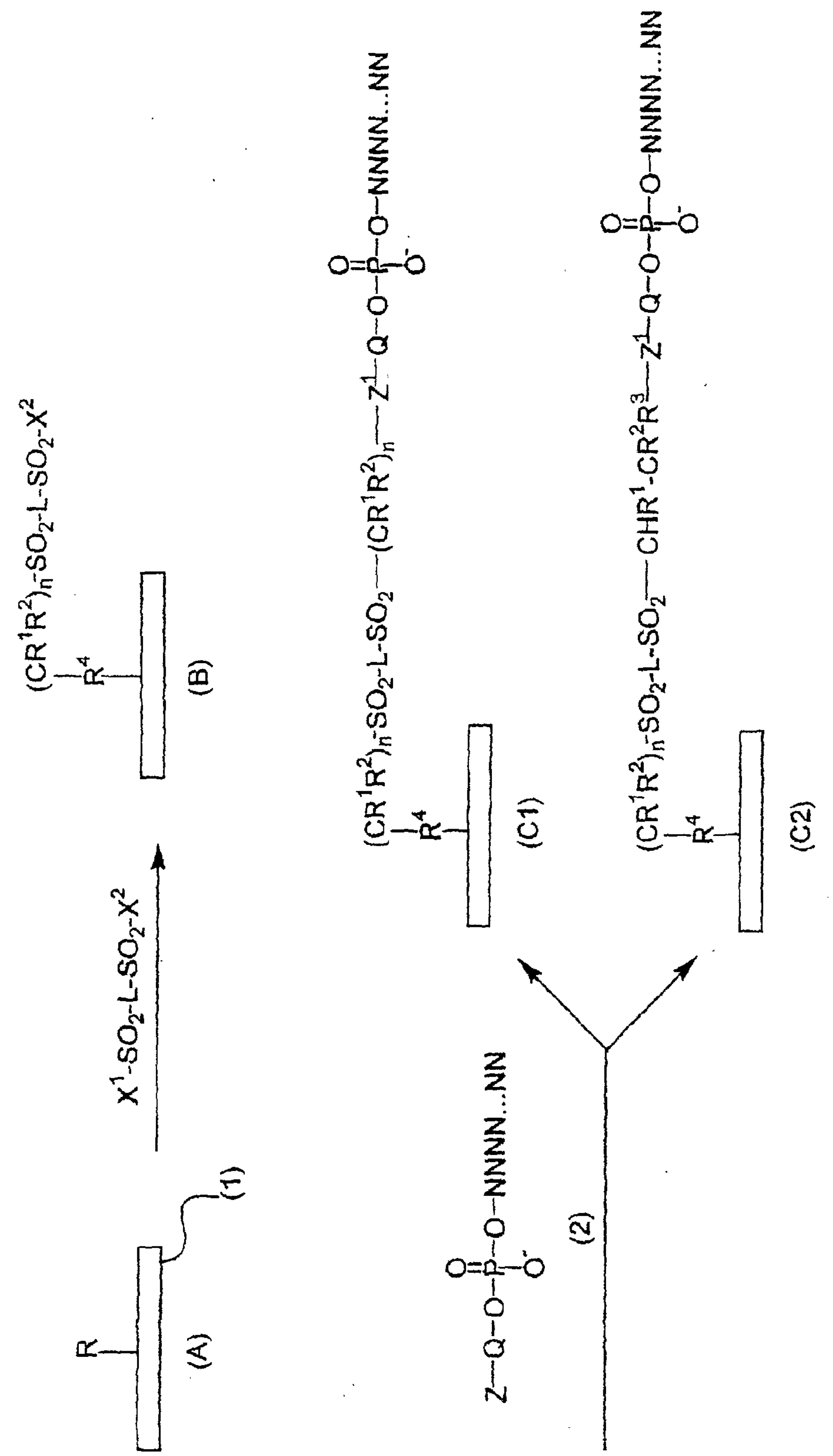

Fig.2

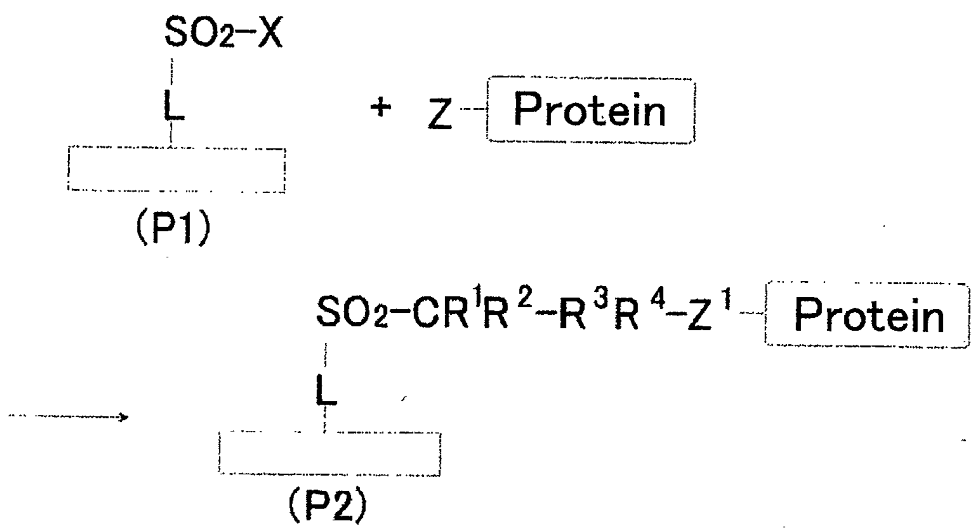

Fig.3

SEQUENCE LISTING

<110> Fuji Photo Film Co.Ltd.,

<120> REACTIVE SOLID SUPPORT AND DNA FRAGMENT DETECTION TOOL

<130> FA2143A

<160> 2

<210> 1

<211> 22

<212> DNA

<213> Artificial DNA

<400> 1

ctagtctgtg aagtgtctga tc    22

<210> 2

<211> 40

<212> DNA

<213> Artificial DNA

<400> 2

tcctccatgt ccggggagga tctgacactt caaggtctag    40